# EUROPEAN PATENT SPECIFICATION

(11) **EP 2 432 501 B1**
(45) Date of publication and mention of the grant of the patent: **14.12.2016**
(21) Application number: 10778504.0
(22) Date of filing: 21.05.2010
(51) Int. Cl.: A61K 39/112

(54) **SECRETION-RELATED BACTERIAL PROTEINS FOR NLRC4 STIMULATION**
SEKRETIONSBEZOGENE BAKTERIELLE PROTEINE ZUR NLRC4-STIMULATION
PROTÉINES BACTÉRIENNES ASSOCIÉES À DES SÉCRÉTIONS POUR STIMULER NLRC4

(30) Priority: 22.05.2009 US 180762 P
(43) Date of publication of application: 28.03.2012
(73) Proprietor: The Institute for Systems Biology, Seattle, Washington 98109-5234 (US)
(72) Inventor: MIAO, Edward, A., Seattle, WA 98133 (US); ADEREM, Alan, A., Seattle, WA 98122 (US)
(74) Representative: Uexküll & Stolberg
(86) International application number: PCT/US2010/035855
(87) International publication number: WO 2010/135704

(56) References cited:
- WO-A1-02/085933
- WO-A1-2007/101337
- WO-A1-2008/025171
- WO-A2-2007/044406
- WO-A2-2009/130618
- US-A1- 2002 098 236
- US-A1- 2006 034 871
- US-A1- 2007 116 711
- US-A1- 2009 074 817
- MIAO EDWARD A ET AL: "Pseudomonas aeruginosa activates caspase 1 through Ipaf", PROCEEDINGS OF THE NATIONAL ACADEMY OF SCIENCES OF THE UNITED STATES OF AMERICA, vol. 105, no. 7, February 2008 (2008-02), pages 2562-2567, XP002690063, ISSN: 0027-8424
- EDWARD A MIAO ET AL: "Cytoplasmic flagellin activates caspase-1 and secretion of interleukin 1[beta] via Ipaf", NATURE IMMUNOLOGY, vol. 7, no. 6, 30 April 2006 (2006-04-30), pages 569-575, XP055013907, ISSN: 1529-2908, DOI: 10.1038/ni1344
- EDWARD A MIAO ET AL: "Innate Immune Detection of Bacterial Virulence Factors Via the NLRC4 Inflammasome", JOURNAL OF CLINICAL IMMUNOLOGY, KLUWER ACADEMIC PUBLISHERS-PLENUM PUBLISHERS, NE, vol. 30, no. 4, 27 March 2010 (2010-03-27) , pages 502-506, XP019822459, ISSN: 1573-2592
- KONJUFCA ET AL.: 'Immunogenicity of recombinant attenuated Salmonella enterica serovar Typhimurium vaccine strains carrying a gene that encodes Eimeria tenella antigen S07.' INFECT. IMMUN. vol. 76, no. 12, December 2008, pages 5745 - 5753
- SCHROEDER ET AL.: 'Molecular pathogenesis of Shigella spp.: controlling host cell signaling, invasion, and death by type III secretion.' CLIN. MICROBIOL. REV. vol. 21, no. 1, January 2008, pages 134 - 156, XP055079440
- LOTTER ET AL. ORAL VACCINATION WITH RECOMBINANT YERSINIA ENTEROCOLITICA EXPRESSING HYBRID TYPE III PROTEINS PROTECTS GERBILS FROM AMEBIC LIVER ABSCESS. vol. 72, no. 12, December 2004, pages 7318 - 7321, XP008164882
- MIAO ET AL.: 'Innate immune detection of the type III secretion apparatus through the NLRC4 inflammasome.' PROC. NATL. ACAD. SCI. USA. vol. 107, no. 7, 16 February 2010, pages 3076 - 3080, XP055013946

## Description

### Technical Field

The invention is directed to compositions that stimulate an immune response in the cytosol by interaction with NLRC4/Ipaf. The active components of these compositions are polypeptides that form the rod component (rod protein) of the type III secretion system (T3SS) apparatus found in many gram negative bacteria.

### Background Art

The mammalian innate immune system uses Toll-like receptors (TLRs) and Nod-like receptors (NLRs) to detect microbial components during infection. Often these molecules work in concert; for example, the TLRs can stimulate the production of the pro-forms of the cytokines IL-1β and IL-18, whereas certain NLRs trigger their subsequent proteolytic processing via caspase-1. Only the processed cytokines are secreted and biologically active. Gram negative bacteria utilize type III secretion systems (T3SS) to deliver virulence factors to the cytosol of host cells, where they modulate cell physiology to favour the pathogen. It is advantageous to the host directly to detect the T3SS thus circumventing the need to respond to the injected virulence factors themselves on a case by case basis.

It has previously been shown that cytosolic flagellin activates caspase-1 via NLRC4/Ipaf. Miao, E. A., et al., PNAS (2008) 105:2562-2567. In *S. typhimurium,* flagellin is promiscuously secreted by the SPI-1 T3SS because of conservation between the flagellar apparatus and the virulence-associated secretion system. Flagellin-independent activation of IL-1β secretion that requires expression of the *prgHIJK* operon that encodes essential structural components of the SPI-1 T3SS apparatus has been observed. Miao, E. A., et al., Nat. Immunol. (2006) 7:569-575.

It has now been found that the rod protein of the T3SS secretion system of SPI-1 of *S*. *typhimurium* (PrgJ) activates NLRC4/Ipaf to effect an innate immune response, and that homologs of this protein have similar effects.

### Disclosure of the Invention

The invention provides immunogenic compositions that activate NLRC4/Ipaf that are comprised of effective portions of the PrgJ protein of *S. typhimurium* or relevant portions of T3SS homologs from other bacteria, *i.e.,* the immunomodulatory T3SS polypeptides (IT3SSP). The ability to stimulate NLRC4 is described herein based on structural comparisons and confirmed as exemplified below.

Thus, in one aspect, the invention is directed to compositions that interact with NLRC4 to engender an innate immune response which compositions comprise as an active ingredient, a polypeptide that comprises at least a portion of the T3SS rod protein of gram negative bacterium or an expression system for its production. As the interaction with the receptor is intracellular, the composition must include a means for introducing the relevant polypeptide into the cytosol. Thus, the peptide may be coupled to an amino acid sequence that promotes transfection, included in a formulation for transfection of protein, or an expression system for it may be included in a viral- or bacterial-based composition that introduces the polypeptide into the cells.

Accordingly, the invention relates to a composition that is effective to introduce into the cells of a subject an immunomodulatory T3SS polypeptide (IT3SSP) or a nucleic acid that encodes said IT3SSP for use in a method of eliciting an innate immune response in said subject.

The composition of the invention comprises:
(a) an isolated replication-competent virus or a viral vector which comprises an expression system for a nucleotide sequence that encodes said IT3SSP; or
(b) an isolated bacterial strain that has been modified to contain an expression system for an exogenous IT3SSP; or
(c) a eukaryotic parasitic microorganism that has been modified to contain an expression system for said IT3SSP; or
(d) a fusion protein comprising said IT3SSP fused to an amino acid sequence that facilitates cell penetration into the cells of said subject; or
(e) a naked nucleic acid encoding said IT3SSP; or
(f) said IT3SSP and a protein transfection agent, said transfection agent optionally coupled to said IT3SSP; or
(g) a nucleic acid molecule encoding said IT3SSP and a nucleic acid transfection agent; or
(h) combinations thereof; and
wherein said IT3SSP consists of 39 amino acids at least 95% identical with the C-terminal 39 amino acids in PrgJ as set forth in SEQ ID NO:3 1, or at least 95% identical with a portion of a rod protein consisting of the C-terminal 39 amino acids of a sequence selected from SEQ ID NOs:7-28 and which IT3SSP is verified to activate NLRC4.

In yet another aspect the invention provides a method of screening for an NLRC4 ligand, agonist or antagonist, comprising:
(a) contacting an NLRC4 polypeptide with a candidate compound in the presence of an IT3SSP under conditions wherein binding of said IT3SSP to said NLRC4 polypeptide produces a resultant signal;
(b) determining the production of said resultant signal in the presence of said candidate compound; and
(c) comparing said resultant signal in the presence of said candidate compound with a resultant signal in the absence of said candidate compound, wherein a difference between said resultant signals in the presence and absence of said candidate compound indicates that said compound is an NLRC4 ligand, agonist or antagonist,
wherein said IT3SSP consists of 39 amino acids at least 95% identical with the C-terminal 39 amino acids in PrgJ as set forth in SEQ ID NO: 31, or at least 95% identical with a portion of a rod protein consisting of the C-terminal 39 amino acids of a sequence selected from SEQ ID NOs:7-28 and which IT3SSP is verified to activate NLRC4.

In still another aspect the invention refers to a nucleic acid molecule which comprises an expression system which expression system comprises a nucleotide sequence encoding an IT3SSP operably linked to a heterologous promoter, wherein said IT3SSP consists of 39 amino acids at least 95% identical with the C-terminal 39 amino acids in PrgJ as set forth in SEQ ID NO: 31 or at least 95% identical with a portion of a rod protein consisting of the C-terminal 39 amino acids of a sequence selected from SEQ ID NOs:7-28; and which IT3SSP is verified to activate NLRC4.

In still another aspect the invention refers to a composition comprising a viral vector or attenuated bacterium, virus, parasite or host cell that comprises the nucleic acid molecule as defined above; or a fusion protein which fusion protein comprises IT3SSP coupled to an antigen and/or to an amino acid sequence that facilitates cell penetration; or IT3SSP and a protein transfection agent, said transfection agent optionally coupled to said IT3SSP; or a nucleic acid that encodes IT3SSP and a nucleic acid transfection agent, wherein said IT3SSP consists of 39 amino acids at least 95% identical with the C-terminal 39 amino acids in PrgJ as set forth in SEQ ID NO: 31 or at least 95% identical with a portion of a rod protein consisting of the C-terminal 39 amino acids of a sequence selected from SEQ ID NOs:7-28 and which IT3SSP is verified to activate NLRC4.

Finally, the invention relates to the use of the above composition for use in a method of raising an immune response.

### Brief Description of the Drawings

Figure 1a shows diagrammatically the polymerized flagellin (FliC) and the T3SS system showing the positions of PrgI and PrgJ in T3SS.
Figure 1b shows stereochemical comparisons of FIiC, PrgI and PrgJ.
Figures 1c and 1d show a sequence comparison of various rod proteins at positions homologous to PrgJ, starting at the position shown in parentheses at the left.
Figure 1e shows a comparison of the amino acid sequences from position 455 to the C-terminus of FliC with the positions downstream of position 61 of PrgJ.
Figures 2a and 2b show the results of transfecting bone marrow macrophage (BMM) with FliC, PrgJ or PrgI. Figure 2a shows these results as a function of amount of protein supplied and Figure 2b is a Western blot comparing the results obtained with BMM from wildtype mice with those from BMM obtained from NLRC4 null mice.
Figures 3a-3d show the results of a retroviral lethality screen using green fluorescent protein (GFP) expression as an indicator of cell viability. Control vectors which express only GFP result in viable cells both in wildtype and NLRC4 null cells (Figures 3a and 3b). However, cells transfected with vectors that produce GFP and PrgJ do not survive as shown in Figure 3c although NLRC4 null cells do survive (Figure 3d).
Figure 4 is a graph showing the ability of FliC but not PrgJ to stimulate TLR5.
Figure 5 shows the effect of mutations at the C-terminus of PrgJ on the ability to effect IL-1β secretion.
Figure 6 is a depiction of the phylogenetic tree of T3SS rod proteins encoded by various species of bacteria.
Figures 7a and 7b are graphs showing detection of the T3SS system by macrophages, which respond by secreting IL-1β. This response requires NLRC4. Figure 7a shows various levels of suppression of IL-1β secretion when the flagellin-based or T3SS-based mechanisms are abated. Figure 7b shows that only in wildtype cells is the effect of native EPEC exerted.
Figures 8a and 8b show a comparison of the effectiveness of *S. typhimurium* SPI-1 and SPI-2 systems on IL-1β secretion.

### Modes of Currying Out the Invention

Evidence has been found for the likelihood that some features of the T3SS apparatus may, similarly to flagellin, interact with analogous receptors based on structural similarity comparisons.

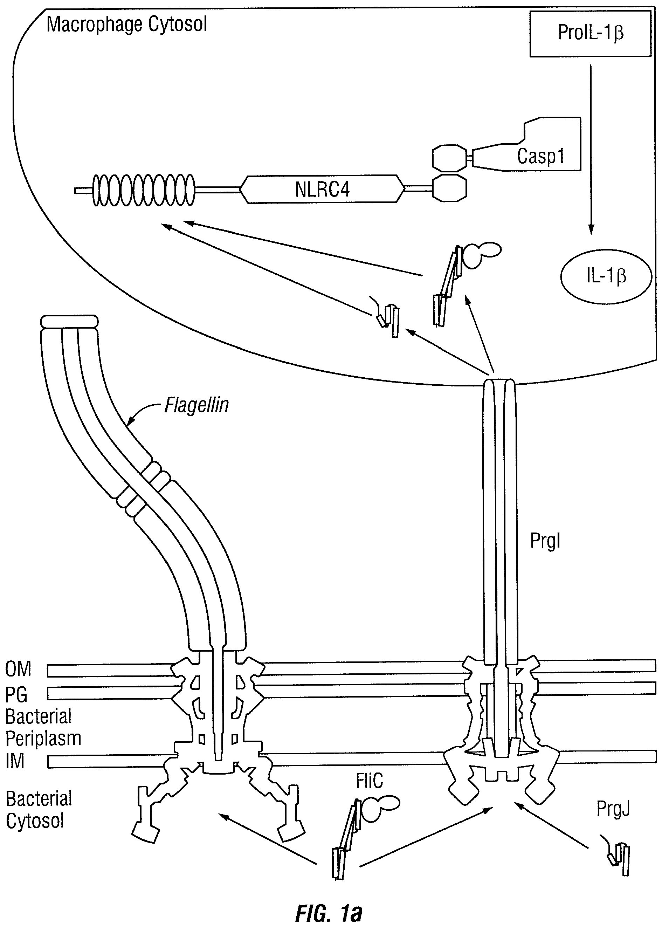

Flagellin (FliC) polymerizes into a hollow tube that forms the flagellar filament, and the PrgJ and PrgI of the T3SS similarly polymerize into a hollow tube and form the rod and needle of the *S. typhimurium* SPI-1 T3SS. (See Figure 1a.) Furthermore, like FliC, both PrgI and PrgJ are secreted by the SPI-1 T3SS.

Given that many of the detectable sequence-based relationships between PrgJ and other proteins of interest are weak and likely too distant to be properly detected based purely on sequences, protein structure prediction was used to search for 3D-structure similarities between flagellin, PrgJ and other T3SS apparatus proteins. Published three-dimensional structures for flagellin (FliC) and PrgI are available (Yonekura, K., et al., Nature (2003) 424:643-650; and Wang, Y., et al., J. Mol. Biol. (2007) 371:1304-1314) and two approaches to predict the structure of PrgJ were employed.

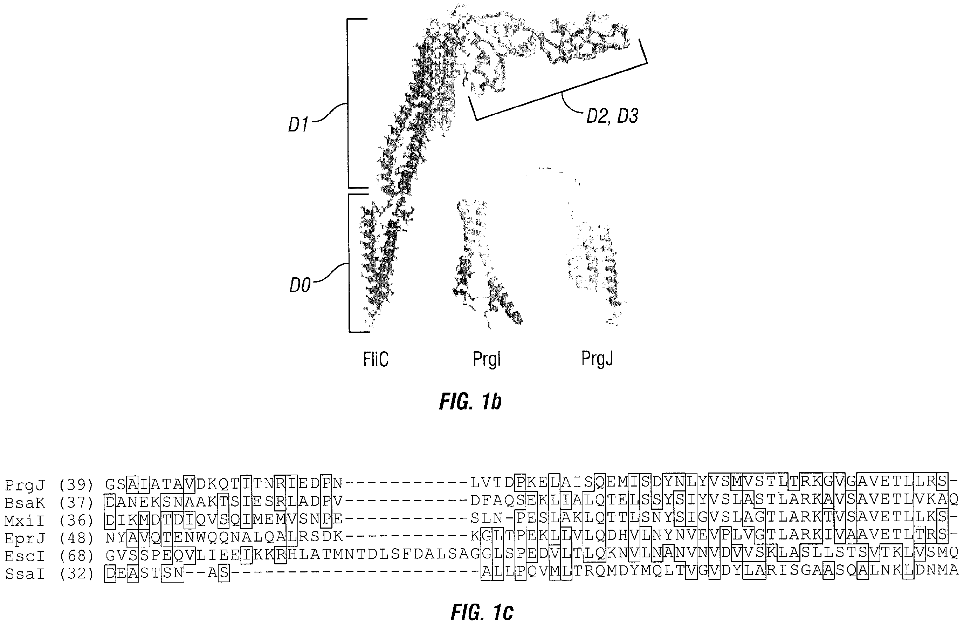

One approach to predict the structure of PrgJ employs the Human Proteome Folding (HPF) pipeline (Bonneau, R., et al., Genome Biol. (2004) 5:R52 (*supra*); and Bonneau, R., et al., J. Struct. Biol. (2001) 134:186-190 (*supra*)) and the other employs the 3D-jury fold-recognition server (Kajan, L., et al., BMC Bioinformatics (2007) 5:304). Multiple fold recognition methods, employed by both methods, found strong hits to the experimentally determined structure of other known type III secretion apparatus proteins PrgI (PDB code 2jow) and BsaL (PDB code 2g0u, the PrgI homolog from *B. pseudomallei,* Zhang, L., et al., J. Mol. Biol. (2006) 359:322-330). Further, both methods found similar simple three-helix folds, suggesting that the fold identification is correct. Models resulting from FFAS (Jaroszewski, L., et al., Nucleic Acids Res. (2005) 33:W284-288) hits to 2g0u chain A and 2jow were refined using Modeller (Fiser, A., et al., Protein Sci. (2000) 9:1753-1773) resulting in confident structure predictions for residues 23-98 of PrgJ. The 23 N-terminal residues were not well aligned to the template structures and the N-terminal residues of 2jow and 2g0u are not well resolved in the experimental structure, suggesting that the N-terminal region of PrgJ and other related proteins are not ordered in solution. These residues could be ordered upon polymerization or upon interaction with other T3SS apparatus components. As shown in Figure 1b, the D0 portion of FliC located at the C-terminus and the 3-dimensional structure of PrgJ appear similar.

### Immunomodulatory T3SS Polypeptides

The compositions of the invention useful to generate an NLRC4-stimulated innate immune response must provide an "immunomodulatory T3SS polypeptide" to the cytosol of an affected cell. Thus, the immunomodulatory T3SS polypeptide (abbreviated herein "IT3SSP") must either be generated intracellularly from encoding nucleic acids introduced into the cell, such as through viral or bacterial vectors or contained in the cell by virtue of other means of transfection, or transfected into the cell by means of fusion to, or presence of assistor proteins or other transfection agents that effect protein transit into the cytosol.

The subjects for which the compositions of the invention are useful in eliciting innate immune responses are generally vertebrate organisms including mammals and birds. The subjects may be human subjects or veterinary subjects such as livestock or household pets. The subjects may be laboratory animal model systems such as mice, rats, rabbits and the like and may include primates.

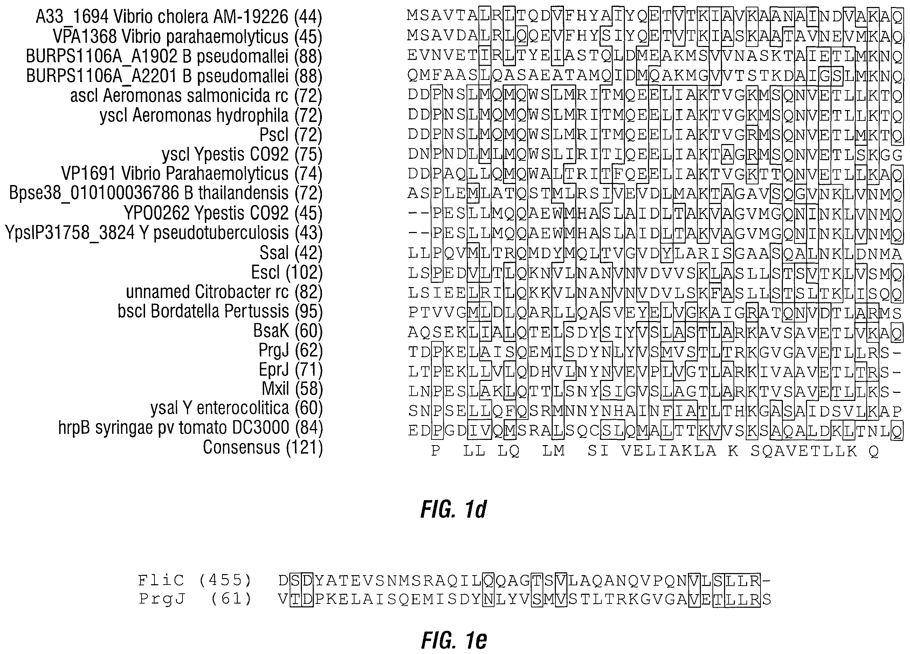

The IT3SSP itself is based on at least the sequence shown at the 39 C-terminal amino acids of the *S. typhimurium* peptide PrgJ (Figure 1e) and the corresponding regions in other naturally occurring bacterial T3SS rod proteins. As shown in Figure 1e, the corresponding region of flagellin is not homologous. The relevant sequences of other bacterial rod proteins are shown in Figures 1c and 1d. Some modifications of these sequences can be made so long as the ability of the peptide to activate NLRC4 is not destroyed. The seven amino acids at the C-terminus of PrgJ must be present or only conservative substitutions made at these positions. In particular, the hydrophobic amino acids valine, leucine, isoleucine, and methionine may only be replaced by other members of this group when they occur at positions within the carboxy-terminal seven amino acids of the rod protein.

The corresponding portions to the critical 39 amino acid sequence of PrgJ for various homologs of PrgJ are shown in Figure 1d, where a consensus sequence is also shown. Additional IT3SSP's may be identified by alignment with the cortical sequence of the rod proteins shown in Figure 1d. The sequences for many rod proteins are available in the art as follows. (Those explicitly set out in the present application are included for completeness):

| **Bacterial Species** | **Gene name** | **GenBank accession number** |
|---|---|---|
| Salmonella typhimurium | prgJ | (NP_461793) |
| Salmonella typhimurium | ssaI | (NP_460373) |
| Escherichia coli | EprJ | (NP_289414) |
| Escherichia coli | EscI | (CAX18737) |
| Escherichia coli | unnamed | (NP_290272) |
| Burkholderia pseudomallei | bsaK | (YP_001076126) |
| Burkholderia pseudomallei | hrpB2 | (YP_001076234) |
| Burkholderia pseudomallei | rpB2 2 | (YP_001075936) |
| Burkholderia thailandensis | bsaK | (YP_439021) |
| Burkholderia thailandensis | hrpB2 | (YP_438945) |
| Burkholderia thailandensis | unnamed | (ZP_02468967) |
| Xanthomonas campestris pv campestris | hrpB2 | (NP_636606) |
| Yersinia pseudotuberculosis | hrpB | (YP_001402778) |
| Pseudomonas aeruginosa | pscI | (NP_250413) |
| Yersinia pestis | yscI | (NP_395192) |
| Yersinia pestis | KIM | (NP_667858) |
| Yersinia pestis | CO92 | (YP_002345343.1) |
| Yersinia enterocolitica | ysaI | (AAK84118) |
| Shigella flexneri | mxiI | (NP_085301) |
| Sodalis glossinidius | unnamed | (YP_454974) |
| Sodalis glossinidius | ysaI | (AAS66834) |
| Providencia stuartii | unnamed | (EDU57885) |
| Proteus mirabillis | unnamed | (YP_002152401) |
| Chromobacterium violaceum | prgJ | (NP 902091) |
| Chromobacterium violaceum | unnamed | (NP_902257) |
| Edwardsiella ictaluri | esaI | (ABC60062) |
| Schnewanella baltica | unnamed | (Y_001554693) |
| Yersinia intermedia | unnamed | (ZP_00833570) |
| Citrobacter rodentium | unnamed | (AAL06365) |
| Photorhabdus luminescens | IscI | (AAO18028) |
| Aeromonas salmonicida | ascI | (YP_001144273) |
| Aeromonas hydrophila | yscI | (AAV30248) |
| Vibrio campbellii | unnamed | (ZP_02195914) |
| Vibrio parahaemolyticus | unnamed | (NP_798070) |
| Vibrio parahaemolyticus | unnamed | (NP_800878) |
| Vibrio harveyi | unnamed | (YP_001444902) |
| Hahella chejuensis | unnamed | (YP_434431) |
| Bordatella pertussis | bscI 1 | (NP_880892) |
| Bordatella pertussis | bscI 2 | (CAC79571) |
| Pseudomonas syringae pv maculicola | unnamed | (AAQ20007) |
| Pseudomonas syringae pv tomato | unnamed | (NP_791210) |
| Erwinia tasmaniensis | unnamed | (YP_001906476) |
| Pectobacterium carotovorum | unnamed | (AAQ73919) |
| Pseudomonas viridiflava | unnamed | (AAT96321) |
| Stappia aggregata | unnamed | (ZP_01550008) |
| Pseudomonas syringae | unnamed | (YP_274722) |
| Myxococcus xanthus | unnamed | (YP_633791) |
| Sodalis glossinidius | unnamed | (YP_455757) |
| Pseudomonas aeruginosa | pscF | (NP_250410) |
| Burkholderia thailandensis | fliE2 | (YP_438372) |

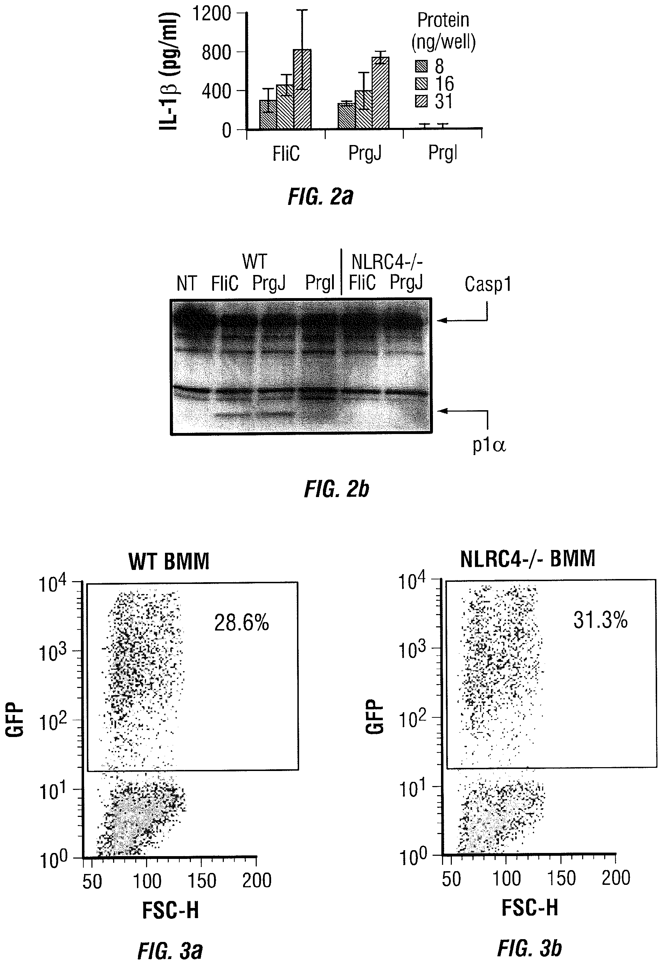

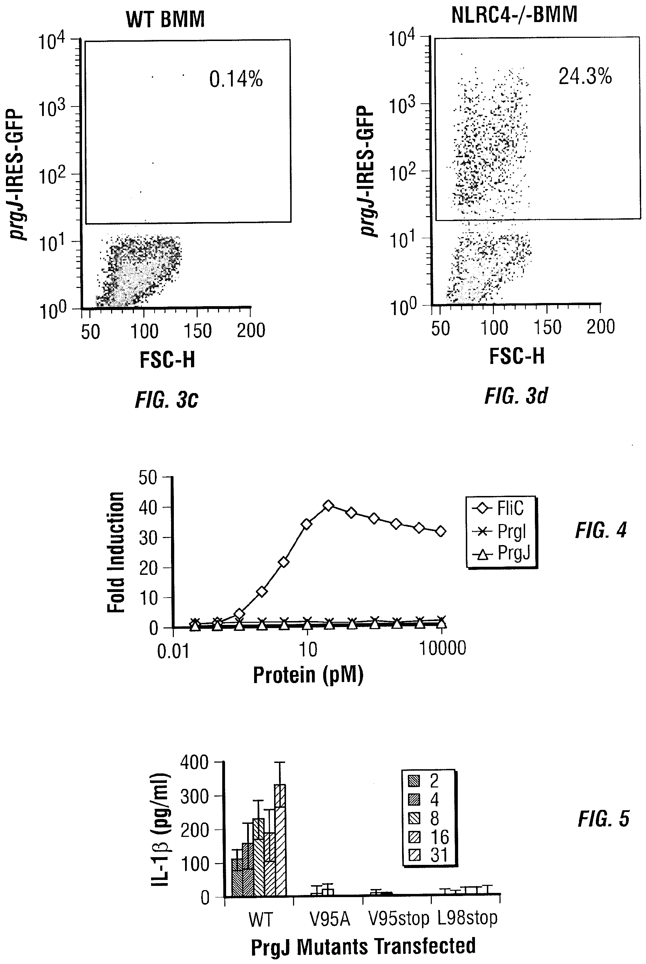

Rod proteins identified in this way will have homology to the family in the 39 amino acid block spanning the consensus sequence PxxLLxLQxxLMxxSIxVELIAKLAxKxSQAVETL LKxQ, however, as few as 12, 13, 14 or 15 matches to this consensus are permitted for rod proteins, as 12 is the case with PrgJ. Experimentally, such candidate rod proteins can readily be verified to be activators of NLRC4 by purification of recombinant protein (note that amino-terminal purification tags are permitted, but carboxy-terminal tags will ablate NLRC4 detection), delivery to the cytosol of LPS primed macrophages, and analysis of IL-lb secretion. This result can be verified using the retroviral lethality screen as depicted in Figure 3.

These tests can easily be performed by the skilled artisan using the consensus sequence for guidance in the design of synthetic homologs that include the essential features of the consensus sequence. Design of such synthetic homologs is also informed by the necessity to terminate the sequence at either the Q shown in the consensus sequence at the C-terminus or at the immediately preceding X. Further extensions beyond the C-terminus will inactivate the IT3SSP. Further, it is understood that the region closer to the C-terminus of the consensus sequence is more critical than the upstream portion.

Thus, IT3SSP's may have precisely the same amino acid sequence as that corresponding to the above portion of PrgJ in a native T3SS rod protein such as those shown in Figure 1d or may deviate in inconsequential ways from such sequences. In general, the relevant corresponding sequences are conserved among bacterial species as will be apparent from the full length rod sequences available in GenBank in relation to the consensus sequence. Substitutions, especially substitutions of non-critical residues in these regions, may be tolerated and embodiments of these regions with such substitutions are included within the scope of the invention.

In an alternative definition, variants of a native bacterial T3SS rod protein sequence are included within the definition of "immunomodulatory T3SS polypeptide" as long as the activation of NLRC4 is preserved and as long as the overall amino acid sequence of the relevant 39 mer portion is at least 85% or 95% (or 97% or 99%) identical to the 39 C-terminal amino acids of PrgJ shown in Figure 1e or to the corresponding portion of other rod proteins, especially T3SS rod proteins. The IT3SSP may be further extended at the N-terminus, but not at the C-terminus as noted above. For example, a full-length rod protein can be included as an IT3SSP. Thus, an IT3SSP within the scope of the invention is a protein which contains a 39 C-terminal amino acid sequence of the aforementioned percentage identities to PrgJ or native homolog with an arbitrary number of N-terminal extensions containing, for example, 10, 20 or 30 or more amino acids.

The percent identity between two amino acid or nucleotide sequences can be determined using the algorithm of E. Meyers and W. Miller (Cabios (1989) 4:11-17) which has been incorporated into the ALIGN program (version 2.0), using a PAM120 weight residue table, a gap length penalty of 12 and a gap penalty of 4.

The IT3SSP's of the invention can also be defined in terms of the nucleotide sequences that encode them. Thus, the IT3SSP's of the invention include those encoded by polynucleotides that hybridize under specified stringency conditions to polynucleotides that encode at least the regions conserved based on the consensus sequence shown in Figure 1d of native flagellin proteins or based on other rod sequences found in GenBank. Thus, the IT3SSP's include those encoded by polynucleotides that hybridize to these reference nucleotide sequences, or to their complements, under medium stringency or high stringency. Guidance for performing hybridization reactions can be found in Ausubel, *et al.,* (1998, *supra*), Sections 6.3.1-6.3.6. Medium stringency refers to hybridizing in 6 x SSC at about 45°C, followed by one or more washes in 0.2 x SSC, 0.1% SDS at 60°C. High stringency conditions refer to hybridizing in 6 x SSC at about 45°C, followed by one or more washes in 0.2 x SSC, 0.1% SDS at 65°C.

In some embodiments, an IT3SSP is encoded by a polynucleotide that hybridizes to a disclosed nucleotide sequence under "very high" stringency conditions, which refers to hybridizing 0.5 M sodium phosphate, 7% SDS at 65°C, followed by one or more washes at 0.2 x SSC, 1% SDS at 65°C.

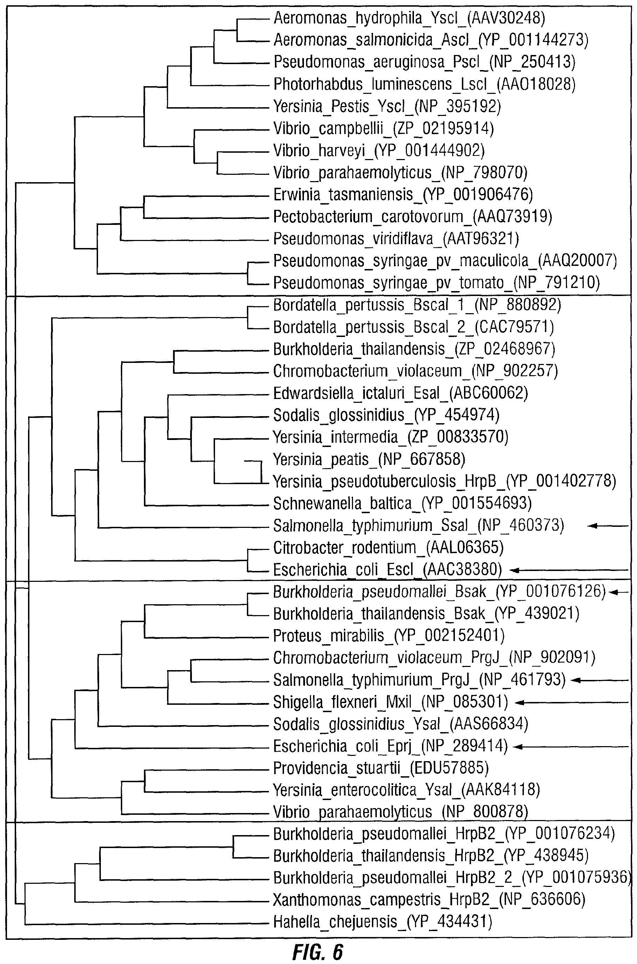

Thus, given the guidance provided, IT3SSP can be designed based on known sequences of rod proteins, such as those available from GenBank and those depicted in Figure 1d along with the knowledge that the sequences close to the C-terminus are the most critical and that further extensions at the C-terminus are not effective in retaining activity. Further guidance can be obtained from Figure 6 which shows the relatedness of various bacterial species which provide putative homologs to PrgJ.

### Compositions Useful in the Method of the Invention

As noted above, the IT3SSP's are effective only intracellularly, and thus compositions useful in the methods of the invention must sometimes contain additional components which result in the intracellular presence of these polypeptides. While in some instances, naked DNA simply encoding the IT3SSP may be effective for administration to a subject to effect an innate immune response, more commonly aids to cellular transfection are included. Generally, these may be separate transfection agents operative with proteins or nucleic acids or these agents fused to the IT3SSP, or fusion proteins with cell transfection enhancers may be used, as well as vectors that themselves facilitate cellular entry, such as bacteria and viruses.

Thus IT3SSP chimeric or fusion proteins may be used for generating an immune response in a mammal. IT3SSP may be linked to at least a peptide that enhances cell penetration; for example, a transduction domain or cell-penetrating peptide, such as those described for the HIV transcription factor tat or the *Drosophila* transcription factor Antennapedia, among others (*see* Green, et al., TRENDS in Pharmacological Sciences (2003) 24:213-215; Chauhan, et al., J. Control Release (2007) 117:148-162; and Vives, et al., J. Biol Chem (1997) 272:16010-16017. The inclusion of a transduction domain facilitates uptake of an IT3SSP by affected cells. In certain embodiments, the cell-penetrating peptide may comprise the amino acid sequence RKKRRQR, which is derived from HIV tat.

Further, certain post-translational modifications may be used to deliver IT3SSP containing proteins to the cytosol. The myristoyl group is a naturally occurring posttranslational modification that serves to target cytoplasmic proteins to intracellular membranes, such that myristoylation of a polypeptide leads to membrane targeting, however, myristoylation has also been shown to deliver extracellular protein to the cytosol (Nelson, et al., Biochemistry (2007) 46:14771-14781). The enzyme N-myristoyltransferase catalyzes the covalent attachment of myristate to the N-terminus of various proteins according to the presence of an appropriate sequence motif (Maurer-Stroh, et al., J. Mol Biol. (2002) 317:523-540). Accordingly, the IT3SSP may be modified with an appropriate protein myristoylation motif to allow the attachment of a myristoyl group to the N-terminus of the IT3SSP during production *in vitro.* Subsequent delivery of this protein to a subject will result in the delivery of IT3SSP to the cytosol and activation of NLRC4.

The heterologous amino acid sequences fused to the IT3SSP may also include one or more antigens for which an adaptive immune response is desired. Such antigens include antigens representative of infectious agents, including viruses, bacteria and parasites; antigens that represent endogenous targets, such as tumor-associated antigens; and any other sequence to which an immune response is desired. Suitable viral and bacterial antigens are associated with the diseases against which the compositions may be targeted as described in detail below. The nature of tumor-associated antigens is also well known in the art, and such antigens are often based on individual expression in endogenous tumors.

Formulations may also be prepared which contain materials that effect transfection of proteins into the cytosol. Commercially available transfection agents for proteins include BIOPORTER^{®} which is a cationic lipid comprised of trifluoroacetylated lipopolyamine and dioleoylphosphatidylethanolamine. Another such agent is TRANS IT^{®} which is a histone-based polyamine. These formulations may also, of course, include antigens to which an antigen-specific response is desired. The IT3SSP's may also be covalently coupled to such transfection agents or, as noted above, derivatized with a transfection agent such as myristic acid, which may also be considered a protein transfection agent.

The IT3SSP may also be generated intracellularly by use of an expression system. Methods for introducing such expression systems into cells include infection by viral vectors or bacterial systems which provide means for crossing the cellular membrane. In some cases, naked DNA is effective. In addition, expression systems or encoding nucleic acids may be introduced using suitable formulations that effect transfection, such as those used commonly to introduce DNA or RNA into cells.

As to viral vectors, an isolated, replication-competent or infectious virus that encodes and expresses an IT3SSP upon entering and infecting a target cell, may be used. The virus may be attenuated, such that it replicates within a host but does not cause a significantly pathological condition.

Expression of the IT3SSP from a viral vector releases the polypeptide into the cytosol of infected cells, thus activating NLRC4 and may also do so when the IT3SSP is fused to a viral protein, or other protein such as an antigen.

In certain embodiments, the replication-competent virus is selected from optionally attenuated Adenoviridae, Caliciviridae, Picornoviridae, Herpesviridae, Hepadnaviridae, Filoviridae, Flaviviridae, Retroviridae, Orthomyxoviridae, Papovaviridae, Parvoviridae, Poxviridae, Reoviridae, Togaviridae, and Influenzae. The insertion of the expression system for IT3SSP or the nucleotide sequence itself may result in this attenuation.

A viral vector may be replication-competent upon administration to a mammal, as described herein, or it may be competent only for a single round of infection upon administration. Typically, the polynucleotide sequences encoding the IT3SSP and the desired antigen are operably linked to one or more promoter sequences. In certain embodiments, the IT3SSP and the desired polypeptide antigen may form a fusion or chimeric protein. As such, a viral vector delivery system comprising an endogenously expressed IT3SSP may be utilized to generate an enhanced immune response to any desired antigen.

Live attenuated bacteria may also be used to deliver the expression system. The bacteria comprise an exogenous nucleotide sequence that encodes an IT3SSP, wherein the exogenous nucleotide sequence is operably linked to a bacterial promoter.

The IT3SSP will be expressed such that it gains access to the mammalian cytosol. This may be accomplished by fusion to classical bacterial secretion signals for pathogens that reside within the cytosol (Mycobacterium, Listeria, *Shigella).* Alternatively, the IT3SSP will be delivered by the bacterial T3SS virulence secretion system, as in some cases rod components already contain T3SS secretion signals (Salmonella, Shigella, *Campylobacter*). Another alternative delivery method can be engineered by adding type IV secretion system (T4SS) signals to the T3SS rod for delivery by organisms that express T4SS (*Helicobacter*).

The modified bacteria will elicit both an innate response due to the interaction of the IT3SSP with the NLRC4 as well as an enhanced adaptive response to the antigens present on the bacteria.

If the bacterial strain used in the infection expresses certain virulence factors or other secretion apparatuses, then these may facilitate the transport of IT3SSP into the cytosol of host cells, thereby activating NLRC4. Examples include the type III secretion system (such as found in *Salmonella)* and the type IV secretion system (such as found in *Legionella),* as well as others. IT3SSP can be translocated from the bacterial cytosol to the host cytosol by these two systems without the addition of heterologous secretion signals,. Thus, for bacteria that express a type III secretion system, but do not express IT3SSP, IT3SSP can be expressed in the bacteria and translocated into the host cytosol. Exemplary bacteria for which this may be useful are *Salmonella* spp and *Yersinia pestis.*

Live bacterial vaccines include bacterial strains that replicate in a host, so that the vaccine may elicit an immune response similar to that elicited by the natural infection. A live bacterial vaccine may be attenuated, meaning that its disease-causing capacity is minimized or eliminated by biological or technical manipulations. Typically, a live bacterial vaccine is neither underattenuated, *i.e.*, retaining even limited pathogenicity, nor overattenuated, *i.e.*, being no longer infections enough to be an effective vaccine. Live bacterial vaccines usually elicit both humoral immunity as well as cellular immunity. Live bacterial vaccines containing an exogenous IT3SSP, described herein, elicit increased innate immune responses that will promote more vigorous humoral and cellular immune responses in turn.

The bacterial strain may be one that does not contain an endogenous IT3SSP gene, or has been modified so as not to produce endogenous IT3SSP or may also contain an endogenous IT3SSP-encoding nucleotide sequence.

Eukaryotic parasitic organisms may also be used to generate the IT3SSP, wherein the parasitic organism comprises an exogenous nucleotide sequence that encodes an IT3SSP, operably linked to a promoter. Examples of parasitic organisms include, but are not limited to, *Entemoeba histolytica, Necator americanus, Ancylostoma duodenale, Leishmania, Plasmodium falciparum, P. vivax, P. ovale, P. malariae), Schistosoma mansoni, S. haematobium, S. japonicum, Onchocerca volvulus, Trypanosoma cruzi,* and *Dracunculus medinensis.*

Pharmaceutical formulations typically comprise a pharmaceutically acceptable carrier or excipient in combination with the IT3SSP compositions of the invention. Vaccine compositions may comprise an additional pharmaceutically acceptable adjuvant.

The pharmaceutical and vaccine formulations may be administered according to any appropriate route of administration, including, but not limited to, inhalation, intradermal, transdermal, intramuscular, topically, intranasal, subcutaneous, direct injection, and formulation.

Compositions of the present invention may include suspensions of the active agents as provided herein (*e*.*g*., viruses or bacteria), which may be prepared in water suitably mixed with a surfactant, such as hydroxypropylcellulose. Dispersions may also be prepared in glycerol, liquid polyethylene glycols, and mixtures thereof and in oils. Under ordinary conditions of storage and use, these preparations contain a preservative to prevent the growth of undesirable microorganisms. The pharmaceutical forms suitable for injectable use include sterile aqueous solutions or dispersions and sterile powders for the extemporaneous preparation of sterile injectable solutions or dispersions In all cases the solution form should be sterile and fluid to the extent that easy syringability exists. It should be stable under the conditions of manufacture and storage and should be preserved against the contaminating action of unwanted microorganisms, such as bacteria and fungi unrelated to the vaccine agent provided therein. The carrier can be a solvent or dispersion medium containing, for example, water, ethanol, polyol (*e*.*g*., glycerol, propylene glycol, and liquid polyethylene glycol, and the like), suitable mixtures thereof, and/or vegetable oils. Proper fluidity may be maintained, for example, by the use of a coating, such as lecithin, by the maintenance of the required particle size in the case of dispersion and by the use of surfactants. In certain embodiments, in which live bacteria are not included in the composition, the prevention of the action of undesired microorganisms can be brought about by various antibacterial and antifungal agents, for example, parabens, chlorobutanol, phenol, sorbic acid, thimerosal, and the like. In many cases, it will be preferable to include isotonic agents, for example, sugars or sodium chloride. Prolonged absorption of the injectable compositions can be brought about by the use in the compositions of agents delaying absorption, for example, aluminum monostearate and gelatin.

In general, suitable formulations are described in Remington's Pharmaceutical Sciences, latest edition, Mack Publishing Co., Easton, PA

### Methods of Treatment/Diseases

The present invention contemplates utilizing the compositions provided herein for treating or reducing the risk of acquiring a wide variety of disease or conditions, including infectious diseases such as viral infections, bacterial infections, and parasitic infections, in addition to conditions caused by pathologically aberrant cells, such as degenerative conditions or cancer.

In general, the IT3SSP's, when administered to a subject in compositions which effect the entry of the polypeptides into the cytosol or the generation of the polypeptides in the cytosol, result in an enhanced innate immune response. This innate immune response intensifies an adaptive immune response that is antigen-specific. Thus, the formulations of the invention may include or be administered along with an antigen against which an immune response is desired. Further, the desired response may be to an endogenous antigen, such as a tumor-associated antigen, in which case inclusion of an antigen in the composition may not be necessary to elicit an adaptive response.

With respect to immune response to diseases, examples of viral infectious diseases or agents include, but are not limited to, Hepatitis A, Hepatitis B, Hepatitis C, Hepatitis E, Caliciviruses associated diarrhea, Rotavirus diarrhea, Haemophilus influenzae B pneumonia and invasive disease, Influenza, measles, mumps, rubella, Parainfluenza associated pneumonia, Respiratory syncytial virus (RSV) pneumonia, Severe Acute Respiratory Syndrome (SARS), Human papillomavirus, Herpes simplex type 2 genital ulcers, HIV/AIDS, Dengue Fever, Japanese encephalitis, Tick-borne encephalitis, West-Nile virus associated disease, Yellow Fever, Epstein-Barr virus, Lassa fever, Crimean-Congo haemorrhagic fever, Ebola haemorrhagic fever, Marburg haemorrhagic fever, Rabies, Rift Valley fever, Smallpox, leprosy, upper and lower respiratory infections, poliomyelitis, among others described elsewhere herein.

Examples of bacterial infections disease or agents include, but are not limited to, *Bacillus antracis, Borellia burgdorferi, Brucella abortus, Brucella canus, Brucella melitensis, Brucella suis, Campylobacter jejuni, Chlamydia pneumoniae, Chlamydia psitacci, Chlamydia trachomatis, Clostridium botulinum, C. difficile, C. perfringens, C. tetani, Corynebacterium diphtheriae (i.e.,* diphtheria), *Ereterococcus, Escherichia coli, Haemophilus influenza, Helicobacter pylori, Legionella pneumophila*, *Leptospira, Listeria monocytogenes, Mycobacterium leprae, M. tuberculosis, Mycoplasma pneumoniae, Neisseria gonorrhea, N. meningitidis, Pseudomonas aeruginasa, Rickettsia recketisii, Salmonella typhi, S. typhimurium, Shigella sonnei, Staphylococcus aureus, S*. *epidermidis, S*. *saprophyticus, Streptococcus agalactiae, S. pneumoniae, S*. *pyogenes, Treponema pallidum, Vibrio cholera, Yersinia pestis, Bordatella pertussis,* and *otitis media (e.g.,* often caused by *Streptococcus pneumoniae, Haemophilus influenzae,* or *Moraxella catarrhalis*), among others described elsewhere herein.

Certain embodiments contemplate methods of treating or reducing the risk of a pathogenic parasitic infection or parasitic disease in a mammal, comprising administering to the mammal a composition comprising an isolated eukaryotic parasitic organism, wherein the parasitic organism comprises an exogenous nucleotide sequence that encodes an IT3SSP, and wherein the exogenous nucleotide sequence is operably linked to a promoter. Examples of parasitic infectious diseases include, but are not limited to, Amebiasis (*e.g., Entemoeba histolytica*), Hookworm Disease (*e.g.,* nematode parasites such as *Necator americanus* and *Ancylostoma duodenale*), Leishmaniasis, Malaria (four species of the protozoan parasite *Plasmodium; P. falciparum, P. vivax, P. ovale*, and *P. malariae*), Schistosomiasis (parasitic Schistosoma; *S. mansoni*, *S. haematobium,* and *S. japonicum), Onchocerca volvulus* (River blindness), *Trypanosoma cruzi* (Chagas disease/American sleeping sickness), and *Dracunculus medinensis*, lymphatic filariasis.

The methods provided herein may also be used to treat or reduce the risks associated with conditions characterized by "pathologically aberrant cells," such as cancer or degenerative conditions. For example, certain embodiments contemplate methods of treating a cancerous or degenerative condition (*i.e*., a condition characterized by "pathologically aberrant cells), comprising administering to the mammal a composition comprising an isolated replication-competent virus or a replication-incompetent virus (*i.e.*, competent for a single round of infection only), wherein the replication-competent virus comprises a nucleotide sequence that encodes an IT3SSP, and wherein the virus comprises a nucleotide sequence that encodes a desired antigen. In certain embodiments, the desired antigen is associated with a cancer cell, such as a tumor cell, but is not significantly associated with a normal cell. For example, the cancer or tumor cell may express a characteristic antigen on its cell surface, which could provide a target for immunotherapy using a vaccine as provided herein. For example, 5T4 antigen expression is widespread in malignant tumors throughout their development, and is found in tumors such as colorectal, ovarian, and gastric tumors. 5T4 expression is used as a prognostic aid in these cases, since it has very limited expression in normal tissue, and, therefore, represents a desired antigen for use with the methods provided herein. It is believed that stimulating an enhanced immune response against an antigen associated with a cancer cell, such as by stimulating an NLRC4-mediated cellular response, will induce an immune response, such as an cellular immune response, against the cancer or tumor cell, thereby helping to destroy the cancer or tumor cell.

Examples of cancers or tumors that may be treated according to the present invention include, but are not limited to, prostate cancer, lung cancer, colorectal cancer, bladder cancer, cutaneous melanoma, pancreatic cancer, leukemia, breast cancer, endometrial cancer, non-Hodgkin's lymphoma, ovarian cancer, malignant melanoma, renal cell carcinoma, thyroid cancer, skin cancer (nonmelanoma).

The following examples are offered to illustrate but not to limit the invention.

### Preparation A

### General Methods for Infection

Overnight *S. typhimurium* and *E. coli* were back diluted 1:40 or 1:50 and grown at 37°C with shaking for 3 or 4 hours to induce SPI-1 (*Salmonella-based*) or LEE (*E. coli* based) T3SS expression, respectively before infecting BMM. SPI-2 T3SS expression is induced after several hours in macrophages, thus for SPI-2 infections, overnight S. *typhimurium* which do not express SPI-1 T3SS were used. BMM from C57BL/6 (Jackson Labs) or NLRC4 deficient mice were primed with 50 ng/ml LPS for 3-4 hours before infections to induce proIL-1β expression. *S*. *typhimurium* infections were performed for one hour after centrifugation as described (Miao, E. A., et al., Nat. Immunol. (2006) 7:569-575) with longer infections followed by treatment with 15 ug/ml gentamicin. *E. coli* infections were performed with five minute centrifugation at 233 xg followed by one hour incubation at 37°C, then addition of gentamicin to limit extracellular replication and three more hours incubation before determination of IL-1β secretion.

### Example 1

### Intracellular Effect of PrgJ

The effect of purified FliC, PrgI and PrgJ on It-1β secretion when delivered intracellularly was examined. The proteins were purified using Talon™ beads (Clontech) taking advantage of a histidine tag on each.

Each of these polypeptides was delivered using the protein transfection reagent Pro-Feet™ as described by Miao, E. A., et al., Proc. Natl. Acad. Sci. USA (2008) 105:2562-2567 to the cytosol of bone marrow macrophage (BMM) from C57BL/6 mice (Jackson Labs) primed with 50 ng/ml LPS for 3-4 hours before transfections to induce proIL-1β expression. IL-1β secretion was determined by ELISA one hour later. The results are shown in Figure 2a. As shown, using 8, 16, or 31 ng of protein per ml, both FliC and PrgJ elicited a dose-dependent amount of IL-1β up to 800 pg/ml at the highest protein concentration. PrgI, on the other hand, did not elicit a response.

In an additional experiment, BMM from wildtype or NLRC4 null mice were transfected with purified FliC, PrgJ or PrgI protein for one hour and caspase-1 processing into p20 and p10 was determined as described by Miao, E., A., et al., Nat. Immunol. (2006) 7:569-575 referenced *supra.* The resulting Western blot is shown in Figure 2b. Only wildtype cells responded positively to PrgJ or FliC; cells from NLRC4-/- mice did not respond. None of the cells responded to PrgI. While p10 was generated by both FliC and PrgJ in wildtype cells, this effect did not occur when the cells were lacking NLRC4.

### Example 2

### Retroviral Lethality Screen

This assay is based on the fact that NLRC4-dependent caspase-1 activation in BMM results in pyroptosis, a rapid nonapoptotic form of programmed cell death. BMM from C57BL/6 mice or mice that had been modified to delete NLRC4 were employed and compared. Transgenic retroviruses, obtained by cloning the relevant sequences into pMXsIG (described in Kitamura, et al., Int. J. Hematol (1998) 67:351:359), which contains an IRES-GFP element to track retroviral infection, where used. Retroviruses were generated in ecotropic phoenix cells (ATCC) as described by Miao, E. A., *et al,,* (2006) *supra* and spinfections to induce infection were performed on BMM which had been harvested 2-4 days previously. GFP expressing cells were determined by flow cytometry two days after infection.

As shown in Figure 3, GFP expression was observed both in wildtype and NLRC4 null macrophage transduced with vectors simply expressing GFP (Figures 3a and 3b). However, for cells transfected with vectors containing PrgJ-IRES-GFP, only NLRC4 null cells survived to produce GFP (Figures 3c and 3d). Similar results were obtained with vectors containing FliC-IRES-GFP. (data not shown) This verifies that pyroptosis induced by FliC or PrgJ is dependent on the presence of NLRC4.

### Example 3

### Stimulation of TLR5

The ability of FliC, PrgI and PrgJ to stimulate TLR5 was tested as described in Smith, K., et al., Nature Immunol. (2003) 4:1247-1253. As shown in Figure 4, while flagellin protein successfully stimulates TLR5, neither PrgI nor PrgJ do so.

### Example 4

### Effect of C-Terminal Truncation

The experiment described in Example 1 was repeated using PrgJ having truncations within the last seven amino acids. As shown in Figure 5a, while wildtype, as predicted, showed a dose-dependent ability to effect secretion of IL-1β, mutants wherein valine at position 95 was replaced by alanine or where valine 95 or leucine 98 was followed by a stop codon, were completely unable to effect secretion of this cytokine. Similarly, PrgJ truncated after the leucine at position 98 did not confer lethality on wildtype (or NLRC4 null) BMM when tested according to the procedure of Example 2. For wildtype cells 28.4% show GFP positive cells and for NLRC4 null cells 32.4% show GFP positive cells by flow cytometry.

### Example 5

### Effect of PrgJ Homologs

PrgJ homologs are found in most T3SS and fall into four large clades as shown in Figure 6. Several homologs of PrgJ, such as BsaK (*Burkholderia pseudomallei*), EprJ (enterohemorrhagic *Escherichia coli,* EHEC), EscI (present in both EHEC, which encodes two T3SS, and enteropathogenic *E. coli,* EPEC), and MxiI (*Shigella flexneri*), share varying sequence similarity to PrgJ (Figure 1c). In this figure, residues that are identical in three of the six sequences are denoted in black and similar residues are noted in grey. Using the retroviral lethality screen of Example 2, BsaK, EprJ, EscI, and MxiI were shown to activate NLRC4 as shown in Table 1.

**Table 1**

| | WT | NLRC4^{-/-} |
|---|---|---|
| GFP | 46.9% | 45.0% |
| *prgJ* | 0.5% | 28.0% |
| *bsaK* | 0.9% | 28.0% |
| *eprJ* | 0.9% | 32.5% |
| *escI* | 0.2% | 33.7% |
| *mxiI* | 0.9% | 23.5% |
| *prgJ* L98stop | 28.4% | 32.4% |
| *ssaI* | 41.8% | 43.1% |

As shown, although GFP-producing cells transfected with control vectors are shown by flow cytometry at levels of 46.9% and 45.0% in wildtype and NLRC4 null cells, respectively, vectors encoding all of these homologs effected pyroptosis in wildtype but not in NLRC4 null cells. *S. flexneri* has previously been shown to induce NLRC4 dependent caspase-1 activation in macrophages independent of flagellin (Suzuki, T., et al., PLoS Pathog. (2007) 3:e111), although the mechanism was not defined; the results above suggest that the response shown by Suzuki, *et al.,* is via MxiI recognition. The results also show that NLRC4 can promote IL-1β secretion in response to the enteropathogenic *E. coli* (EPEC) T3SS encoded by the locus of enterocyte effacement (LEE).

### Example 6

### Confirmation of PrgJ Activity

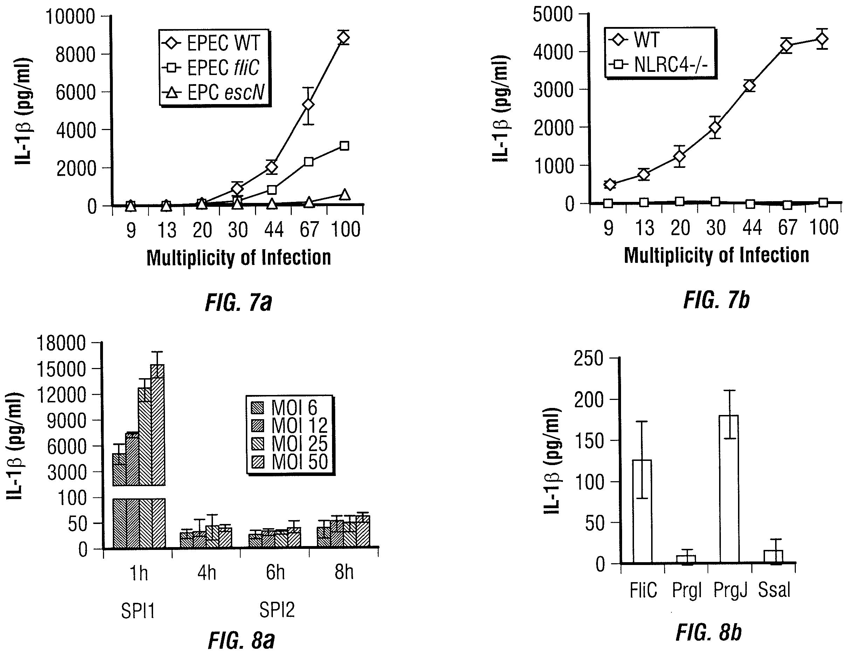

BMM were infected with wild type enteropathogenic *E. coli* (EPEC) or EPEC strains carrying mutations in flagellin (*fliC*) or the T3SS *escN* (which ablates all T3SS translocation activity). In agreement with the *S*. *typhimurium* results, IL-1β secretion was triggered by both a flagellin-dependent and a flagellin-independent pathway as shown in Figure 7a. As shown, wildtype *E. coli* containing intact EPEC were highly effective in causing secretion of IL-1p whereas *E. coli* with mutations in flagellin (EPEC *fliC*) or in EPEC *escN* had diminished or virtually no capacity to effect secretion.

Figure 7b shows both pathways require expression of NLRC4 in macrophages and T3SS in bacteria. Figure 7b shows that only wildtype BMM were successfully caused to secrete IL-1β when treated with *E. coli* with wildtype EPEC whereas NLRC4 null BMM did not secrete IL-1β in response to such infection. In each case, the infection was conducted at the multiplicity of infection shown on the X-axis for one hour and gentamicin was added to the medium and infection continued for three hours. IL-1β secretion was determined by ELISA as described above. Thus, NLRC4 responds to T3SS activity via the detection of two conserved protein agonists: flagellin and the rod protein of the T3SS apparatus.

### Example 7

### SsaI Evades NLRC4

*S. typhimurium* encodes two T3SS, SPI-1 and SPI-2, that promote different aspects of virulence. While *S*. *typhimurium* expressing SPI-1 activated IL-1β secretion in macrophages, SPI-2 expressing bacteria did not. These results are shown in Figure 8a. The experiment of Example 1 was conducted using various levels of infection by *S. typhimurium* expressing either SPI-1 or SPI-2. Even after eight hours at high levels of infection, SPI-2 failed to cause secretion of IL-1β. On the other hand, after only one hour, *S. typhimurium* expressing SPI-1 were quite successful.

As noted above, SsaI, the PrgJ homolog in SPI-2 T3SS, was not detected in retroviral lethality experiments. In addition, protein transfection experiments employing SsaI also failed to effect secretion of IL-1β as shown in Figure 8b.

### Example 8

### Pathogenesis of S. typhimurium SPI-1

For competitive index assays, mice were infected intraperitoneally with 1 x 10⁵ *S. typhimurium* equally divided between vector control (kanamycin resistant) and experimental strain (ampicillin resistant). Colony forming units were enumerated two days later from the spleen and the ratio of control to experimental strain was determined. The control vector contained PrgI under control of the SPI-2 promoter while experimental vectors express PrgJ using the same promoter.

*S. typhimurium* ectopically expressing PrgJ were efficiently cleared from the mouse spleen, whereas ectopic expression of PrgI had no effect on virulence as shown in Table 2. The clearance of bacteria was dependent upon NLRC4, as equivalent numbers of WT and PrgJ expressing bacteria were recovered from NLRC4 deficient mice. Thus, evasion of NLRC4 by the SsaI rod component of the SPI-2 T3SS apparatus is critical for *S. typhimurium* virulence.

**Table 2**

| Mice | kan | amp | Ratio (amp/kan) |
|---|---|---|---|
| WT | vector | pSPI2 *prgI* | 1.62 ± 0.87 |
| WT | vector | pSPI2 *pryJ* | 0.01 ± 0.01 |
| NLRC44-/- | vector | pSPI2 *prgJ* | 1.50 ± 0.61 |

## Claims

1. A composition effective to introduce into the cells of a subject an immunomodulatory T3SS polypeptide (IT3SSP) or a nucleic acid that encodes said IT3SSP for use in a method of eliciting an innate immune response in said subject, wherein said composition comprises:
(a) an isolated replication-competent virus or a viral vector which comprises an expression system for a nucleotide sequence that encodes said IT3SSP; or
(b) an isolated bacterial strain that has been modified to contain an expression system for an exogenous IT3SSP; or
(c) a eukaryotic parasitic microorganism that has been modified to contain an expression system for said IT3SSP; or
(d) a fusion protein comprising said IT3SSP fused to an amino acid sequence that facilitates cell penetration into the cells of said subject; or
(e) a naked nucleic acid encoding said IT3SSP; or
(f) said IT3SSP and a protein transfection agent, said transfection agent optionally coupled to said IT3SSP; or
(g) a nucleic acid molecule encoding said IT3SSP and a nucleic acid transfection agent; or
(h) combinations thereof; and
wherein said IT3SSP consists of 39 amino acids at least 95% identical with the C-terminal 39 amino acids in PrgJ as set forth in SEQ ID NO:31, or at least 95% identical with a portion of a rod protein consisting of the C-terminal 39 amino acids of a sequence selected from SEQ ID NOs:7-28 and which IT3SSP is verified to activate NLRC4.

2. The composition for use in a method of claim 1, wherein said composition further contains an antigen to which an antigen-specific response is desired.

3. The composition for use in a method of claim 1, wherein the replication-competent virus in (a) is or the viral vector in (a) is derived from *Adenoviridae, Caliciviridae, Picornoviridae, Herpesviridae, Hepadnaviridae, Filoviridae, Flaviviridae, Retroviridae, Orthomyxoviridae, Papovaviridae, Parvoviridae, Poxviridae, Reoviridae, Togaviridae,* or *Influenzae.*

4. The composition for use in a method of claim 1, wherein, in (a) the nucleotide sequence that encodes the IT3SSP is inserted into a nucleotide sequence that encodes a viral polypeptide, or is fused to a nucleotide sequence that encodes an antigen.

5. The composition for use in a method of claim 1, wherein the bacterial strain in (b) does not comprise an endogenous T3SS gene, and the encoded IT3SSP is operably linked to a signal sequence.

6. The composition for use in a method of claim 1, wherein the bacterial strain in (b) is selected from *Mycobacterium tuberculosis, Mycobacterium leprae, Yersinia pestis, Neisseria gonorrhea, Chlamydia trachomatis, Chlamydia pneumoniae, Streptococcus pneumoniae, Staphylococcus aureus,* group A *Streptococcus,* group B *Streptococcus, Neisseria meningiditis, Haemophilus influenzae, Acinetobacter baumannii, Helicobacter pylori* and *Campylobacter jejuni.*

7. The composition for use in a method of claim 1, wherein the fusion protein in (d) further comprises a viral, bacterial or parasite antigen.

8. The composition for use in a method of claim 1, wherein the naked nucleic acid in (g) or the nucleic acid in (e) is fused to a nucleotide sequence that encodes an antigen.

9. The composition for use in a method of claim 1, wherein the IT3SSP in (f) is fused to an antigen.

10. A method of screening for an NLRC4 ligand, agonist or antagonist, comprising:
(a) contacting an NLRC4 polypeptide with a candidate compound in the presence of an IT3SSP under conditions wherein binding of said IT3SSP to said NLRC4 polypeptide produces a resultant signal;
(b) determining the production of said resultant signal in the presence of said candidate compound; and
(c) comparing said resultant signal in the presence of said candidate compound with a resultant signal in the absence of said candidate compound, wherein a difference between said resultant signals in the presence and absence of said candidate compound indicates that said compound is an NLRC4 ligand, agonist or antagonist,
wherein said IT3SSP consists of 39 amino acids at least 95% identical with the C-terminal 39 amino acids in PrgJ as set forth in SEQ ID NO: 31, or at least 95% identical with a portion of a rod protein consisting of the C-terminal 39 amino acids of a sequence selected from SEQ ID NOs:7-28 and which IT3SSP is verified to activate NLRC4.

11. The method of claim 10, wherein said NLRC4 is contained in a cell and said resultant signal is amount of an IL-1β.

12. A nucleic acid molecule which comprises an expression system which expression system comprises a nucleotide sequence encoding an IT3SSP operably linked to a heterologous promoter,
wherein said IT3SSP consists of 39 amino acids at least 95% identical with the C-terminal 39 amino acids in PrgJ as set forth in SEQ ID NO: 31 or at least 95% identical with a portion of a rod protein consisting of the C-terminal 39 amino acids of a sequence selected from SEQ ID NOs:7-28; and which IT3SSP is verified to activate NLRC4.

13. The nucleic acid molecule of claim 12, wherein the heterologous promoter is a viral promoter, a bacterial promoter or a promoter derived from a eukaryotic parasite.

14. A composition comprising a viral vector or attenuated bacterium, virus, parasite or host cell that comprises the nucleic acid molecule of claim 12 or 13; or
a fusion protein which fusion protein comprises IT3SSP coupled to an antigen and/or to an amino acid sequence that facilitates cell penetration; or
IT3SSP and a protein transfection agent, said transfection agent optionally coupled to said IT3SSP; or
a nucleic acid that encodes IT3SSP and a nucleic acid transfection agent,
wherein said IT3SSP consists of 39 amino acids at least 95% identical with the C-terminal 39 amino acids in PrgJ as set forth in SEQ ID NO: 31 or at least 95% identical with a portion of a rod protein consisting of the C-terminal 39 amino acids of a sequence selected from SEQ ID NOs:7-28 and which IT3SSP is verified to activate NLRC4.

15. The composition of any of claims 1-9 or 14 or the nucleic acid of claim 12 or 13 or the method of claim 10 or 11 wherein the IT3SSP is at least 99% identical to the C-terminal 39 amino acids of PrgJ as set forth in SEQ ID NO: 31 or at least 99% identical to a portion of a rod protein consisting of the C-terminal 39 amino acids of a sequence selected from SEQ ID NOs:7-28 and which IT3SSP is verified to activate NLRC4.

16. The composition of claim 14 for use in a method of raising an immune response.

## Patentansprüche

1. Zusammensetzung, wirksam, um in die Zellen eines Subjekts ein immunmodulatorisches T3SS-Polypeptid (IT3SSP), oder eine Nucleinsäure, die das IT3SSP codiert, einzuführen, zur Verwendung in einem Verfahren zum Auslösen einer angeborenen Immunantwort in dem Subjekt,
wobei die Zusammensetzung umfasst:
(a) ein isoliertes Replikations-kompetentes Virus oder einen viralen Vektor, welcher ein Expressionssystem für eine Nucleotidsequenz umfasst, die das IT3SSP codiert; oder
(b) einen isolierten Bakterienstamm, der modifiziert worden ist, um ein Expressionssystem für ein exogenes IT3SSP zu enthalten; oder
(c) einen eukaryotischen parasitischen Mikroorganismus, der modifiziert worden ist, um ein Expressionssystem für das IT3SSP zu enthalten; oder
(d) ein Fusionsprotein, umfassend das IT3SSP, fusioniert mit einer Aminosäuresequenz, die die Zellpenetration in die Zellen des Subjekts erleichtert; oder
(e) eine nackte Nucleinsäure, codierend das IT3SSP; oder
(f) das IT3SSP und ein Protein-Transfektionsmittel, wobei das Transfektionsmittel wahlweise mit dem IT3SSP gekoppelt ist; oder
(g) ein Nucleinsäuremolekül, codierend das IT3SSP und ein Nucleinsäure-Transfektionsmittel; oder
(h) Kombinationen davon; und
wobei das IT3SSP aus 39 Aminosäuren, mindestens 95% identisch mit den C-terminalen 39 Aminosäuren in PrgJ, wie angegeben in SEQ ID NO:31, oder mindestens 95% identisch mit einem Anteil von einem Stabprotein, bestehend aus den C-terminalen 39 Aminosäuren einer Sequenz, ausgewählt aus den SEQ ID NOs:7-28, besteht und von welchem IT3SSP bestätigt ist, dass es NLRC4 aktiviert.

2. Zusammensetzung zur Verwendung in einem Verfahren nach Anspruch 1, wobei die Zusammensetzung weiterhin ein Antigen enthält, für welches eine Antigenspezifische Antwort gewünscht wird.

3. Zusammensetzung zur Verwendung in einem Verfahren nach Anspruch 1, wobei das Replikations-kompetente Virus in (a) ist, oder der virale Vektor in (a) abgeleitet ist von, *Adenoviridae, Caliciviridae, Picornoviridae, Herpesviridae, Hepadnaviridae, Filoviridae, Flaviviridae, Retroviridae, Orthomyxoviridae, Papovaviridae, Parvoviridae, Poxviridae, Reoviridae, Togaviridae* oder *Influenzae.*

4. Zusammensetzung zur Verwendung in einem Verfahren nach Anspruch 1, wobei in (a) die Nucleotidsequenz, die das IT3SSP codiert, in eine Nucleotidsequenz insertiert ist, die ein virales Polypeptid codiert, oder mit einer Nucleotidsequenz fusioniert ist, die ein Antigen codiert.

5. Zusammensetzung zur Verwendung in einem Verfahren nach Anspruch 1, wobei der Bakterienstamm in (b) nicht ein endogenes T3SS-Gen umfasst und das codierte IT3SSP operabel mit einer Signalsequenz verknüpft ist.

6. Zusammensetzung zur Verwendung in einem Verfahren nach Anspruch 1, wobei der Bakterienstamm in (b) aus *Mycobacterium tuberculosis, Mycobacterium leprae, Yersinia pestis, Neisseria gonorrhea, Chlamydia trachomatis, Chlamydia pneumoniae, Streptococcus pneumoniae, Staphylococcus aureus, Gruppe-A-Streptococcus,* Crruppe-B-*Streptococcus, Neisseria meningiditis, Haemophilus influenzae, Acinetobacter baumannii, Helicobacter pylori* und *Campylobacter jejuni* ausgewählt ist.

7. Zusammensetzung zur Verwendung in einemVerfahren nach Anspruch 1, wobei das Fusionsprotein in (d) weiterhin ein virales, bakterielles oder Parasitenantigen umfasst.

8. Zusammensetzung zur Verwendung in einem Verfahren nach Anspruch 1, wobei die nackte Nucleinsäure in (g) oder die Nucleinsäure in (e) mit einer Nucleotidsequenz fusioniert ist, die ein Antigen codiert.

9. Zusammensetzung zur Verwendung in einem Verfahren nach Anspruch 1, wobei das IT3SSP in (f) mit einem Antigen fusioniert ist.

10. Verfahren zum Screening für einen NLRC4-Liganden, -Agonisten oder - Antagonisten, umfassend:
(a) Inkontaktbringen von einem NLRC4-Polypeptid mit einer Kandidatenverbindung in Anwesenheit von einem IT3SSP unter Bedingungen, wobei Bindung von dem IT3SSP an das NLRC4-Polypeptid ein resultierendes Signal erzeugt;
(b) Bestimmen der Erzeugung des resultierenden Signals in Anwesenheit der Kandidatenverbindung; und
(c) Vergleichen des resultierenden Signals in Anwesenheit der Kandidatenverbindung mit einem resultierenden Signal in Abwesenheit der Kandidatenverbindung, wobei ein Unterschied zwischen den resultierenden Signalen in Anwesenheit und Abwesenheit der Kandidatenverbindung anzeigt, dass die Verbindung ein NLRC4-Ligand, -Agonist oder -Antagonist ist,
wobei das IT3SSP aus 39 Aminosäuren, mindestens 95% identisch mit den C-terminalen 39 Aminosäuren in PrgJ, wie angegeben in SEQ ID NO: 31, oder mindestens 95% identisch mit einem Anteil von einem Stabprotein, bestehend aus den C-terminalen 39 Aminosäuren einer Sequenz, ausgewählt aus den SEQ ID NOs:7-28, besteht und von welchem IT3SSP bestätigt ist, dass es NLRC4 aktiviert.

11. Verfahren nach Anspruch 10, wobei das NLRC4 in einer Zelle enthalten ist und das resultierende Signal die Menge von einem In-1β ist.

12. Nucleinsäuremolekül, welches ein Expressionssystem umfasst, welches Expressionssystem eine Nucleotidsequenz, codierend ein IT3SSP, operabel verknüpft mit einem heterologen Promotor, umfasst,
wobei das IT3SSP aus 39 Aminosäuren, mindestens 95% identisch mit den C-terminalen 39 Aminosäuren in PrgJ, wie angegeben in SEQ ID NO: 31, oder mindestens 95% identisch mit einem Anteil von einem Stabprotein, bestehend aus den C-terminalen 39 Aminosäuren einer Sequenz, ausgewählt aus den SEQ ID NOs:7-28, besteht; und von welchem IT3SSP bestätigt ist, dass es NLRC4 aktiviert.

13. Nucleinsäuremolekül nach Anspruch 12, wobei der heterologe Promotor ein viraler Promotor, ein bakterieller Promotor oder ein Promotor, abgeleitet von einem eukaryotischen Parasiten, ist.

14. Zusammensetzung, umfassend einen viralen Vektor oder ein abgeschwächtes Bakterium, ein Virus, einen Parasiten oder eine Wirtszelle, die das Nucleinsäuremolekül nach Anspruch 12 oder 13 umfasst; oder
ein Fusionsprotein, welches Fusionsprotein IT3SSP umfasst, gekoppelt mit einem Antigen und/oder mit einer Aminosäuresequenz, die die Zellpenetration erleichtert; oder
IT3SSP und ein Protein-Transfektionsmittel, wobei das Transfektionsmittel gegebenenfalls mit dem IT3SSP gekoppelt ist; oder
eine Nucleinsäure, die IT3SSP und ein Nucleinsäure-Transfektionsmittel codiert,
wobei das IT3SSP aus 39 Aminosäuren, mindestens 95% identisch mit den C-terminalen 39 Aminosäuren in PrgJ, wie angegeben in SEQ ID NO: 31, oder mindestens 95% identisch mit einem Anteil von einem Stabprotein, bestehend aus den C-terminalen 39 Aminosäuren einer Sequenz, ausgewählt aus den SEQ ID NOs:7-28, besteht und von welchem IT3SSP bestätigt ist, dass es NLRC4 aktiviert.

15. Zusammensetzung nach einem der Ansprüche 1-9 oder 14 oder Nucleinsäure nach Anspruch 12 oder 13 oder Verfahren nach Anspruch 10 oder 11, wobei das IT3SSP mindestens 99% identisch mit den C-terminalen 39 Aminosäuren von PrgJ, wie angegeben in SEQ ID NO: 31, oder mindestens 99% identisch mit einem Anteil von einem Stabprotein, bestehend aus den C-terminalen 39 Aminosäuren einer Sequenz, ausgewählt aus den SEQ ID NOs:7-28, ist und von welchem IT3SSP bestätigt ist, dass es NLRC4 aktiviert.

16. Zusammensetzung nach Anspruch 14 zur Verwendung in einem Verfahren zum Hervorrufen einer Immunantwort.

## Revendications

1. Composition efficace pour introduire dans les cellules d'un sujet un polypeptide T3SS immunomodulateur (IT3SSP) ou un acide nucléique qui code ledit IT3SSP, à utiliser dans un procédé de provocation d'une réponse immune innée chez ledit sujet,
où ladite composition comprend:
(a) un virus isolé compétent à la réplication ou un vecteur viral qui comprend un système d'expression pour une séquence de nucléotides qui code ledit IT3SSP; ou bien
(b) une souche bactérienne isolée qui a été modifiée pour contenir un système d'expression pour un IT3SSP exogène; ou bien
(c) un microorganisme parasitaire eucaryote qui a été modifié pour contenir un système d'expression pour ledit IT3SSP; ou bien
(d) une protéine de fusion comprenant ledit IT3SSP fusionné à une séquence d'acides aminés qui facilite la pénétration cellulaire dans les cellules dudit sujet; ou bien
(e) un acide nucléique nu codant ledit IT3SSP; ou bien
(f) ledit IT3SSP et un agent de transfection de protéines, ledit agent de transfection étant optionnellement couplé audit IT3SSP; ou bien
(g) une molécule d'acide nucléique codant ledit IT3SSP et un agent de transfection d'acides nucléiques; ou bien
(h) des combinaisons de ceux-ci; et
où ledit IT3SSP consiste en 39 acides aminés identiques à au moins 95 % aux 39 acides aminés C-terminaux dans PrgJ tel que présenté dans la SEQ ID NO: 31, ou identiques à au moins 95 % à une partie d'une protéine de la tige consistant en les 39 acides aminés C-terminaux d'une séquence sélectionnée parmi les SEQ ID NO: 7-28 et lequel IT3SSP est confirmé activer NLRC4.

2. Composition à utiliser dans un procédé selon la revendication 1, où ladite composition contient en outre un antigène auquel une réponse antigène-spécifique est désirée.

3. Composition à utiliser dans un procédé selon la revendication 1, où ledit virus compétent à la réplication sous (a) est ou le vecteur viral sous (a) est dérivé de: *Adenoviridae, Caliciviridae, Picornoviridae, Herpesviridae, Hepadnaviridae, Filoviridae, Flaviviridae, Retroviridae, Orthomyxoviridae, Papovaviridae, Parvoviridae, Poxviridae, Reoviridae, Togaviridae* ou *Influenzae.*

4. Composition à utiliser dans un procédé selon la revendication 1, où, sous (a) la séquence de nucléotides qui code l'IT3SSP est insérée dans une séquence de nucléotides qui code un polypeptide viral, ou est fusionnée à une séquence de nucléotides qui code un antigène.

5. Composition à utiliser dans un procédé selon la revendication 1, où la souche bactérienne sous (b) ne comprend pas un gène T3SS endogène, et l'IT3SSP codé est lié d'une manière opérationnelle à une séquence signal.

6. Composition à utiliser dans un procédé selon la revendication 1, où la souche bactérienne sous (b) est sélectionnée parmi *Mycobacterium tuberculoses, Mycobacterium leprae, Yersinia pestis, Neisseria gonorrhea, Chlamydia trachomatis, Chlamydia pneumoniae, Streptococcus pneumoniae, Staphylococcus aureus, Streptococcus* du groupe A, *Streptococcus* du groupe B, *Neisseria meningitidis, Haemophilus influenzae, Acinetobacter baumannii, Helicobacter pylori* et *Campylobacter jejuni*.

7. Composition à utiliser dans un procédé selon la revendication 1, où la protéine de fusion sous (d) comprend en outre un antigène viral, bactérien ou parasitaire.

8. Composition à utiliser dans un procédé selon la revendication 1, où l'acide nucléique nu sous (g) ou l'acide nucléique sous (e) est fusionné à une séquence de nucléotides qui code un antigène.

9. Composition à utiliser dans un procédé selon la revendication 1, où l'IT3SSP sous (f) est fusionné à un antigène.

10. Procédé de criblage pour un ligand, agoniste ou antagoniste de NLRC4, comprenant:
(a) mettre un polypeptide NLRC4 en contact avec un composé candidat en présence d'un IT3SSP dans des conditions dans lesquelles la liaison dudit IT3SSP audit polypeptide NLRC4 produit un signal résultant;
(b) déterminer la production dudit signal résultant en présence dudit composé candidat; et
(c) comparer ledit signal résultant en présence dudit composé candidat à un signal résultant en l'absence dudit composé candidat, où une différence entre lesdits signaux résultants en présence et en l'absence dudit composé candidat indique que ledit composé est un ligand, agoniste ou antagoniste de NLRC4.
où ledit IT3SSP consiste en 39 acides aminés identiques à au moins 95 % aux 39 acides aminés C-terminaux dans PrgJ tel que présenté dans la SEQ ID NO: 31, ou bien identiques à au moins 95 % à une partie d'une protéine de la tige consistant en les 39 acides aminés C-terminaux d'une séquence sélectionnée parmi les SEQ ID NO: 7-28 et lequel IT3SSP est confirmé activer NLRC4.

11. Procédé selon la revendication 10, dans lequel ledit NLRC4 est contenu dans une cellule et ledit signal résultant est la quantité d'une IL-1β.

12. Molécule d'acide nucléique qui comprend un système d'expression, lequel système d'expression comprend une séquence de nucléotides codant un IT3SSP lié d'une manière opérationnelle à un promoteur hétérologue,
où ledit IT3SSP consiste en 39 acides aminés identiques à au moins 95 % aux 39 acides aminés C-terminaux dans PrgJ tel que présenté dans la SEQ ID NO: 31, ou bien identiques à au moins 95 % à une partie d'une protéine de la tige consistant en les 39 acides aminés C-terminaux d'une séquence sélectionnée parmi les SEQ ID NO: 7-28; et lequel IT3SSP est confirmé activer NLRC4.

13. Molécule d'acide nucléique selon la revendication 12, où le promoteur hétérologue est un promoteur viral, un promoteur bactérien ou un promoteur dérivé d'un parasite eucaryote.

14. Composition comprenant un vecteur viral ou une bactérie, un virus, un parasite atténués ou une cellule hôte qui comprend la molécule d'acide nucléique selon la revendication 12 ou 13; ou bien
une protéine de fusion, laquelle protéine de fusion comprend IT3SSP couplé à un antigène et/ou à une séquence d'acides aminés qui facilite la pénétration cellulaire; ou bien
IT3SSP et un agent de transfection de protéines, ledit agent de transfection étant optionnellement couplé audit IT3SSP; ou bien
un acide nucléique qui code IT3SSP et un agent de transfection d'acides nucléiques,
où ledit IT3SSP consiste en 39 acides aminés identiques à au moins 95 % aux 39 acides aminés C-terminaux dans PrgJ tel que présenté dans la SEQ ID NO: 31, ou bien identiques à au moins 95 % à une partie d'une protéine de la tige consistant en les 39 acides aminés C-terminaux d'une séquence sélectionnée parmi les SEQ ID NO: 7-28 et lequel IT3SSP est confirmé activer NLRC4.

15. Composition selon l'une quelconque des revendications 1-9 ou 14 ou l'acide nucléique selon la revendication 12 ou 13 ou le procédé selon la revendication 10 ou 11, où l'IT3SSP est identique à au moins 99 % aux 39 acides aminés C-terminaux de PrgJ tel que présenté dans la SEQ ID NO: 31 ou identique à au moins 99 % à une partie d'une protéine de la tige consistant en les 39 acides aminés C-terminaux d'une séquence sélectionnée parmi les SEQ ID NO: 7-28 et lequel IT3SSP est confirmé activer NLRC4.

16. Composition selon la revendication 14, à utiliser dans un procédé destiné à élever une réponse immune.


### SEQUENCE LISTING

<110> INSTITUTE FOR SYSTEMS BIOLOGY MIAO, Edward A. ADEREM, Alan A.
<120> SECRETION-RELATED BACTERIAL PROTEINS FOR NLRC4 STIMULATION
<130> 655652000440
<140> PCT/US2010/035855
   <141> 2010-05-21
<150> US 61/180,762
   <151> 2009-05-22
<160> 31
<170> FastSEQ for Windows Version 4.0
<210> 1
   <211> 63
   <212> PRT
   <213> Salmonella typhimurium
<220>
   <221> misc_feature
   <222> (1)...(63)
   <223> PrgJ (NP_461793)
<400> 1
   Gly Ser Ala Ile Ala Thr Ala Val Asp Lys Gln Thr Ile Thr Asn Arg
    1               5                  10                  15
   Ile Glu Asp Pro Asn Leu Val Thr Asp Pro Lys Glu Leu Ala Ile Ser
               20                  25                  30
   Gln Glu Met Ile Ser Asp Tyr Asn Leu Tyr Val Ser Met Val Ser Thr
           35                  40                  45
   Leu Thr Arg Lys Gly Val Gly Ala Val Glu Thr Leu Leu Arg Ser
       50                  55                  60
<210> 2
   <211> 64
   <212> PRT
   <213> Burkholderia pseudomallei
<220>
   <221> misc_feature
   <222> (1)...(64)
   <223> BsaK (YP_001076126)
<400> 2
   Asp Ala Asn Glu Lys Ser Asn Ala Ala Lys Thr Ser Ile Glu Ser Arg
    1               5                  10                  15
   Leu Ala Asp Pro Val Asp Phe Ala Gln Ser Glu Lys Leu Ile Ala Leu
               20                  25                  30
   Gln Thr Glu Leu Ser Asp Tyr Ser Ile Tyr Val Ser Leu Ala Ser Thr
           35                  40                  45
   Leu Ala Arg Lys Ala Val Ser Ala Val Glu Thr Leu Val Lys Ala Gln
       50                  55                  60
<210> 3
   <211> 62
   <212> PRT
   <213> Shigella flexneri
<220>
   <221> misc_feature
   <222> (1)...(62)
   <223> MxiI (NP_085301)
<400> 3
   Asp Ile Lys Met Asp Thr Asp Ile Gln Val Ser Gln Ile Met Glu Met
    1               5                  10                  15
   Val Ser Asn Pro Glu Ser Leu Asn Pro Glu Ser Leu Ala Lys Leu Gln
               20                  25                  30
   Thr Thr Leu Ser Asn Tyr Ser Ile Gly Val Ser Leu Ala Gly Thr Leu
           35                  40                  45
   Ala Arg Lys Thr Val Ser Ala Val Glu Thr Leu Leu Lys Ser
       50                  55                  60
<210> 4
   <211> 63
   <212> PRT
   <213> Escherichia coli
<220>
   <221> misc_feature
   <222> (1)...(63)
   <223> EprJ (NP_289414)
<400> 4
   Asn Tyr Ala Val Gln Thr Glu Asn Trp Gln Gln Asn Ala Leu Gln Ala
    1               5                  10                  15
   Leu Arg Ser Asp Lys Lys Gly Leu Thr Pro Glu Lys Leu Leu Val Leu
               20                  25                  30
   Gln Asp His Val Leu Asn Tyr Asn Val Glu Val Pro Leu Val Gly Thr
           35                  40                  45
   Leu Ala Arg Lys Ile Val Ala Ala Val Glu Thr Leu Thr Arg Ser
       50                  55                  60
<210> 5
   <211> 75
   <212> PRT
   <213> Escherichia coli
<220>
   <221> misc_feature
   <222> (1)...(75)
   <223> EscI (CAX18737)
<400> 5
   Gly Val Ser Ser Pro Glu Gln Val Leu Ile Glu Glu Ile Lys Lys Arg
    1               5                  10                  15
   His Leu Ala Thr Met Asn Thr Asp Leu Ser Phe Asp Ala Leu Ser Ala
               20                  25                  30
   Gly Gly Leu Ser Pro Glu Asp Val Leu Thr Leu Gln Lys Asn Val Leu
           35                  40                  45
   Asn Ala Asn Val Asn Val Asp Val Val Ser Lys Leu Ala Ser Leu Leu
       50                  55                  60
   Ser Thr Ser Val Thr Lys Leu Val Ser Met Gln
   65                  70                  75
<210> 6
   <211> 51
   <212> PRT
   <213> Salmonella tymphimurium
<220>
   <221> misc_feature
   <222> (1)...(51)
   <223> SsaI (NP_460373)
<400> 6
   Asp Glu Ala Ser Thr Ser Asn Ala Ser Ala Leu Leu Pro Gln Val Met
   1 5 10 15
   Leu Thr Arg Gln Met Asp Tyr Met Gln Leu Thr Val Gly Val Asp Tyr
   20 25 30
   Leu Ala Arg Ile Ser Gly Ala Ala Ser Gln Ala Leu Asn Lys Leu Asp
   35 40 45
   Asn Met Ala
   50
<210> 7
   <211> 41
   <212> PRT
   <213> Vibrio cholerae
<220>
   <221> misc_feature
   <222> (1)...(41)
   <223> AM-19226 (A33_1694)
<400> 7
   Met Ser Ala Val Thr Ala Leu Arg Leu Thr Gln Asp Val Phe His Tyr
   1 5 10 15
   Ala Ile Tyr Gln Glu Thr Val Thr Lys Ile Ala Val Lys Ala Ala Asn
   20 25 30
   Ala Ile Asn Asp Val Ala Lys Ala Gln
   35 40
<210> 8
   <211> 41
   <212> PRT
   <213> Vibrio parahaemolyticus
<220>
   <221> misc_feature
   <222> (1)...(41)
   <223> VPA1368 (NP_800878)
<400> 8
   Met Ser Ala Val Asp Ala Leu Arg Leu Gln Gln Glu Val Phe His Tyr
   1 5 10 15
   Ser Ile Tyr Gln Glu Thr Val Thr Lys Ile Ala Ser Lys Ala Ala Thr
   20 25 30
   Ala Val Asn Glu Val Met Lys Ala Gln
   35 40
<210> 9
   <211> 41
   <212> PRT
   <213> Burkholderia pseudomallei
<220>
   <221> misc_feature
   <222> (1)...(41)
   <223> BURPS1106A_A1902
<400> 9
   Glu Val Asn Val Glu Thr Ile Arg Leu Thr Tyr Glu Ile Ala Ser Thr
   1 5 10 15
   Gln Leu Asp Met Glu Ala Lys Met Ser Val Val Asn Ala Ser Lys Thr
   20 25 30
   Ala Ile Glu Thr Leu Met Lys Asn Gln
   35 40
<210> 10
   <211> 41
   <212> PRT
   <213> Burkholderia pseudomallei
<220>
   <221> misc_feature
   <222> (1)...(41)
   <223> BURPS1106A_A2201
<400> 10
   Gln Met Phe Ala Ala Ser Leu Gln Ala Ser Ala Glu Ala Thr Ala Met
   1 5 10 15
   Gln Ile Asp Met Gln Ala Lys Met Gly Val Val Thr Ser Thr Lys Asp
   20 25 30
   Ala Ile Gly Ser Leu Met Lys Asn Gln
   35 40
<210> 11
   <211> 41
   <212> PRT
   <213> Aeromonas salmonicida
<220>
   <221> misc_feature
   <222> (1)...(41)
   <223> ascI (YP_001144273)
<400> 11
   Asp Asp Pro Asn Ser Leu Met Gln Met Gln Trp Ser Leu Met Arg Ile
   1 5 10 15
   Thr Met Gln Glu Glu Leu Ile Ala Lys Thr Val Gly Lys Met Ser Gln
   20 25 30
   Asn Val Glu Thr Leu Leu Lys Thr Gln
   35 40
<210> 12
   <211> 41
   <212> PRT
   <213> Aeromonas hydrophila
<220>
   <221> misc_feature
   <222> (1)...(41)
   <223> yscI (AAV30248)
<400> 12
   Asp Asp Pro Asn Ser Leu Met Gln Met Gln Trp Ser Leu Met Arg Ile
    1               5                  10                  15
   Thr Met Gln Glu Glu Leu Ile Ala Lys Thr Val Gly Lys Met Ser Gln
               20                  25                  30
   Asn Val Glu Thr Leu Leu Lys Thr Gln
           35                  40
<210> 13
   <211> 41
   <212> PRT
   <213> Pseudomonas aeruginosa
<220>
   <221> misc_feature
   <222> (1)...(41)
   <223> PscI (NP_250413)
<400> 13
   Asp Asp Pro Asn Ser Leu Met Gln Met Gln Trp Ser Leu Met Arg Ile
    1               5                  10                  15
   Thr Met Gln Glu Glu Leu Ile Ala Lys Thr Val Gly Arg Met Ser Gln
               20                  25                  30
   Asn Val Glu Thr Leu Met Lys Thr Gln
           35                  40
<210> 14
   <211> 41
   <212> PRT
   <213> Yersinia pestis
<220>
   <221> misc_feature
   <222> (1)...(41)
   <223> yscI (NP_395192)
<400> 14
   Asp Asn Pro Asn Asp Leu Met Leu Met Gln Trp Ser Leu Ile Arg Ile
    1               5                  10                  15
   Thr Ile Gln Glu Glu Leu Ile Ala Lys Thr Ala Gly Arg Met Ser Gln
               20                  25                  30
   Asn Val Glu Thr Leu Ser Lys Gly Gly
           35                  40
<210> 15
   <211> 41
   <212> PRT
   <213> Vibrio parahaemolyticus
<220>
   <221> misc_feature
   <222> (1)...(41)
   <223> VP1691 (NP_798070)
<400> 15
   Asp Asp Pro Ala Gln Leu Leu Gln Met Gln Trp Ala Leu Thr Arg Ile
    1               5                  10                  15
   Thr Phe Gln Glu Glu Leu Ile Ala Lys Thr Val Gly Lys Thr Thr Gln
               20                  25                  30
   Asn Val Glu Thr Leu Leu Lys Ala Gln
           35                  40
<210> 16
   <211> 41
   <212> PRT
   <213> Burkholderia thailandensis
<220>
   <221> misc_feature
   <222> (1)...(41)
   <223> Bpse38 (ZP_02468967)
<400> 16
   Ala Ser Pro Leu Glu Met Leu Ala Thr Gln Ser Thr Met Leu Arg Ser
    1               5                  10                  15
   Ile Val Glu Val Asp Leu Met Ala Lys Thr Ala Gly Ala Val Ser Gln
               20                  25                  30
   Gly Val Asn Lys Leu Val Asn Met Gln
           35                  40
<210> 17
   <211> 39
   <212> PRT
   <213> Yersinia pestis
<220>
   <221> misc_feature
   <222> (1)...(39)
   <223> CO92 (YP_002345343.1)
<400> 17
   Pro Glu Ser Leu Leu Met Gln Gln Ala Glu Trp Met His Ala Ser Leu
    1               5                  10                  15
   Ala Ile Asp Leu Thr Ala Lys Val Ala Gly Val Met Gly Gln Asn Ile
               20                  25                  30
   Asn Lys Leu Val Asn Met Gln
           35
<210> 18
   <211> 39
   <212> PRT
   <213> Yersinia pseudotuberculosis
<220>
   <221> misc_feature
   <222> (1)...(39)
   <223> hrpB (YP_001402778)
<400> 18
   Pro Glu Ser Leu Leu Met Gln Gln Ala Glu Trp Met His Ala Ser Leu
    1               5                  10                  15
   Ala Ile Asp Leu Thr Ala Lys Val Ala Gly Val Met Gly Gln Asn Ile
               20                  25                  30
   Asn Lys Leu Val Asn Met Gln
           35
<210> 19
   <211> 41
   <212> PRT
   <213> Salmonella typhimurium
<220>
   <221> misc_feature
   <222> (1)...(41)
   <223> SsaI (NP_460373)
<400> 19
   Leu Leu Pro Gln Val Met Leu Thr Arg Gln Met Asp Tyr Met Gln Leu
    1               5                  10                  15
   Thr Val Gly Val Asp Tyr Leu Ala Arg Ile Ser Gly Ala Ala Ser Gln
               20                  25                  30
   Ala Leu Asn Lys Leu Asp Asn Met Ala
           35                  40
<210> 20
   <211> 41
   <212> PRT
   <213> Escherichia coli
<220>
   <221> misc_feature
   <222> (1)...(41)
   <223> EscI (CAX18737)
<400> 20
   Leu Ser Pro Glu Asp Val Leu Thr Leu Gln Lys Asn Val Leu Asn Ala
    1               5                  10                  15
   Asn Val Asn Val Asp Val Val Ser Lys Leu Ala Ser Leu Leu Ser Thr
               20                  25                  30
   Ser Val Thr Lys Leu Val Ser Met Gln
           35                  40
<210> 21
   <211> 41
   <212> PRT
   <213> Citrobacter rodentium
<220>
   <221> misc_feature
   <222> (1)...(41)
   <223> rc (AAL06365)
<400> 21
   Leu Ser Ile Glu Glu Leu Arg Ile Leu Gln Lys Lys Val Leu Asn Ala
    1               5                   10                  15
   Asn Val Asn Val Asp Val Leu Ser Lys Phe Ala Ser Leu Leu Ser Thr
               20                  25                  30
   Ser Leu Thr Lys Leu Ile Ser Gln Gln
           35                  40
<210> 22
   <211> 41
   <212> PRT
   <213> Bordatella pertussis
<220>
   <221> misc_feature
   <222> (1)...(41)
   <223> bscI 1 (NP_880892)
<400> 22
   Pro Thr Val Val Gly Met Leu Asp Leu Gln Ala Arg Leu Leu Gln Ala
    1               5                   10                  15
   Ser Val Glu Tyr Glu Leu Val Gly Lys Ala Ile Gly Arg Ala Thr Gln
               20                  25                  30
   Asn Val Asp Thr Leu Ala Arg Met Ser
           35                  40
<210> 23
   <211> 41
   <212> PRT
   <213> Burkholderia pseudomallei
<220>
   <221> misc_feature
   <222> (1)...(41)
   <223> BsaK (YP_001076126)
<400> 23
   Ala Gln Ser Glu Lys Leu Ile Ala Leu Gln Thr Glu Leu Ser Asp Tyr
    1               5                   10                  15
   Ser Ile Tyr Val Ser Leu Ala Ser Thr Leu Ala Arg Lys Ala Val Ser
               20                  25                  30
   Ala Val Glu Thr Leu Val Lys Ala Gln
           35                  40
<210> 24
   <211> 40
   <212> PRT
   <213> Chromobacterium violaceum
<220>
   <221> misc_feature
   <222> (1)...(40)
   <223> PrgJ (NP_902091)
<400> 24
   Thr Asp Pro Lys Glu Leu Ala Ile Ser Gln Glu Met Ile Ser Asp Tyr
    1               5                  10                  15
   Asn Leu Tyr Val Ser Met Val Ser Thr Leu Thr Arg Lys Gly Val Gly
               20                  25                  30
   Ala Val Glu Thr Leu Leu Arg Ser
           35                  40
<210> 25
   <211> 40
   <212> PRT
   <213> Escherichia coli
<220>
   <221> misc_feature
   <222> (1)...(40)
   <223> EprJ (NP_289414)
<400> 25
   Leu Thr Pro Glu Lys Leu Leu Val Leu Gln Asp His Val Leu Asn Tyr
    1               5                  10                  15
   Asn Val Glu Val Pro Leu Val Gly Thr Leu Ala Arg Lys Ile Val Ala
               20                  25                  30
   Ala Val Glu Thr Leu Thr Arg Ser
           35                  40
<210> 26
   <211> 40
   <212> PRT
   <213> Shigella flexneri
<220>
   <221> misc_feature
   <222> (1)...(40)
   <223> MxiI (NO_085301)
<400> 26
   Leu Asn Pro Glu Ser Leu Ala Lys Leu Gln Thr Thr Leu Ser Asn Tyr
    1               5                  10                  15
   Ser Ile Gly Val Ser Leu Ala Gly Thr Leu Ala Arg Lys Thr Val Ser
               20                  25                  30
   Ala Val Glu Thr Leu Leu Lys Ser
           35                  40
<210> 27
   <211> 41
   <212> PRT
   <213> Yersinia enterocolitica
<220>
   <221> misc_feature
   <222> (1)...(41)
   <223> ysaI (AAK84118)
<400> 27
   Ser Asn Pro Ser Glu Leu Leu Gln Phe Gln Ser Arg Met Asn Asn Tyr
    1              5                   10                  15
   Asn His Ala Ile Asn Phe Ile Ala Thr Leu Thr His Lys Gly Ala Ser
               20                  25                  30
   Ala Ile Asp Ser Val Leu Lys Ala Pro
           35                  40
<210> 28
   <211> 41
   <212> PRT
   <213> Pseudomonas syringae pv tomato
<220>
   <221> misc_feature
   <222> (1)...(41)
   <223> hrpB (NP_791210)
<400> 28
   Glu Asp Pro Gly Asp Ile Val Gln Met Ser Arg Ala Leu Ser Gln Cys
    1              5                   10                  15
   Ser Leu Gln Met Ala Leu Thr Thr Lys Val Val Ser Lys Ser Ala Gln
               20                  25                  30
   Ala Leu Asp Lys Leu Thr Asn Leu Gln
           35                  40
<210> 29
   <211> 39
   <212> PRT
   <213> Artificial Sequence
<220>
   <223> synthetically constructed consensus sequence
<220>
   <221> VARIANT
   <222> (1)...(39)
   <223> Xaa = any regular amino acid
<400> 29
   Pro Xaa Xaa Leu Leu Xaa Leu Gln Xaa Xaa Leu Met Xaa Xaa Ser Ile
    1              5                   10                  15
   Xaa Val Glu Leu Ile Ala Lys Leu Ala Xaa Lys Xaa Ser Gln Ala Val
               20                  25                  30
   Glu Thr Leu Leu Lys Xaa Gln
           35
<210> 30
   <211> 37
   <212> PRT
   <213> Salmonella typhimurium
<220>
   <221> misc_feature
   <222> (1)...(37)
   <223> FliC (PO6179)
<400> 30
   Asp Ser Asp Tyr Ala Thr Glu Val Ser Asn Met Ser Arg Ala Gln Ile
    1               5                  10                  15
   Leu Gln Gln Ala Gly Thr Ser Val Leu Ala Gln Ala Asn Gln Val Pro
               20                  25                  30
   Gln Asn Val Leu Ser
           35
<210> 31
   <211> 41
   <212> PRT
   <213> Salmonella typhimurium
<220>
   <221> misc_feature
   <222> (1)...(41)
   <223> PrgJ (NP_461793)
<400> 31
   Val Thr Asp Pro Lys Glu Leu Ala Ile Ser Gln Glu Met Ile Ser Asp
    1               5                  10                  15
   Tyr Asn Leu Tyr Val Ser Met Val Ser Thr Leu Thr Arg Lys Gly Val
               20                  25                  30
   Gly Ala Val Glu Thr Leu Leu Arg Ser
           35                  40